# EUROPEAN PATENT APPLICATION

(11) **EP 4 696 773 A1**
(43) Date of publication of application: **18.02.2026**
(21) Application number: 24788153.5
(22) Date of filing: 11.04.2024
(51) Int. Cl.: C12N 5/10, C12N 7/01, C12N 15/85, A61K 9/127, A61K 35/42

(54) **SARNA DELIVERY SYSTEM, PREPARATION METHOD THEREFOR AND USE THEREOF**

(30) Priority: 11.04.2023 CN 202310381377
(71) Applicant: Zhejiang Free Trade Zone Hongan Base Biotechnology Co., Ltd., Zhoushan City, China (Zhejiang) 316013 (CN)
(72) Inventor: ZHANG, Bo, Zhoushan, Zhejiang 316013 (CN); LIU, Junsheng, Zhoushan, Zhejiang 316013 (CN); ZHAN, Shunli, Zhoushan, Zhejiang 316013 (CN)
(74) Representative: M. Zardi & Co S.A.
(86) International application number: PCT/CN2024/087266
(87) International publication number: WO 2024/213051

(57) **Abstract**

Provided is a method for constructing an saRNA microvesicle delivery system, comprising the following steps: (1) designing an saRNA expression vector 1 and a membrane protein expression vector 2; and (2) co-transfecting the expression vector 1 and the expression vector 2 into a host cell; and (3) culturing the host cell, and isolating microvesicles containing an saRNA, the saRNA containing a target gene sequence, a replicon in the saRNA expression vector being an alphavirus replicon, and the microvesicles not containing capsid protein. The system can efficiently deliver the saRNA to cells or *in vivo* so as to realize the expression of a target protein. On this basis, different elements in the delivery system can be further transformed so as to develop a high-efficiency low-immunogenicity modular platform aiming at different diseases and different targets. The delivery system may supplement the toolbox of existing viral and lipid nanoparticle delivery vectors and has good application prospects.

## Description

### CROSS REFERENCE TO THE RELATED APPLICATIONS

The present application claims the priority benefit of Chinese Patent Application No. 2023103813775 filed on April 11, 2023, which is incorporated herein by reference in its entirety.

### TECHNICAL FIELD

The present disclosure belongs to the field of biotechnology, particularly relates to a novel self-amplifying RNA (saRNA) delivery system, which mediates saRNA delivery via microvesicles assembled from viral envelope proteins (E), and achieves targeted expression of target proteins through the self-replication characteristic of saRNA.

### BACKGROUND TECHNOLOGY

After years of technological accumulation, the gene therapy industry has gradually matured, leading the third industrial revolution in biomedicine. Among them, mRNA drugs, as relatively safe preparations, have attracted the attention of researchers both domestically and internationally. They achieve therapeutic purposes by stimulating the immune system to respond or expressing and producing corresponding proteins. Therefore, the expression level of the downstream proteins is closely related to the therapeutic efficacy. One of the drawbacks of traditional mRNA is its instability and susceptibility to degradation, resulting in non-sustained downstream protein expression. If used long-term for disease treatment, patients may need to be injected with large amounts of mRNA preparations, which may increase the toxic and side effects of mRNA therapy. Self-amplifying RNA (saRNA) refers to a nucleic acid sequence that uses its own RNA sequence as a template and undergoes self-replication with the assistance of the RNA polymerase (RdRP) it carries. The primary advantage of saRNA is that it can achieve the same protein expression level as a large amount of traditional mRNA at a very low dose and can continuously produce the target protein. This characteristic can reduce the injection dose and frequency in RNA preparation therapy, while prolonging the therapeutic effect and at the same time reducing potential toxic and side effects that may be caused by RNA and administration vectors.

In addition, the efficient delivery of mRNA preparations has always been one of the important bottlenecks hindering the development of gene drug and gene therapy industries. At present, delivery vectors used in human gene therapy mainly include non-viral vectors such as liposomes, cationic polymers, nanoparticles, etc., and viral vectors such as lentiviruses, adenoviruses and adeno-associated viruses. Non-viral delivery vectors have the characteristics of safety, stability, etc., but they usually have low transfection efficiency, high production costs and obvious side effects. Viral delivery vectors, including two categories, i.e., live viruses and pseudoviruses, are widely used due to their high transduction and expression efficiency. However, live viral vectors, such as influenza viruses, measles viruses, Newcastle disease viruses, and Virus-like vesicles (VLVs), etc., can produce progeny viruses *in vivo,* leading to potential infection risks. They are usually only suitable for gene delivery in the field of vaccines, not for protein drugs, cell and gene therapy, etc., which restricts the development of such vectors in gene delivery. In comparison, pseudoviral vectors have the advantages of only being singly infectious, having no toxic or side effects caused by viral amplification, etc. Their extremely high safety makes pseudoviral vectors have great developmental and application potential as RNA delivery tools, among which the most representative is Virus-replicon particles (VRPs). The VRP delivery system can effectively deliver saRNA into the body through pseudoviral replicon particles with only single infectivity produced by the trans-complementation of alphavirus replicon RNA and alphavirus structural protein RNA. However, the delivery system in the form of VRP also has the fatal defect of generating wild-type viruses through genetic recombination. For example, the packaging system of VEEV-based VRP consists of three parts: VEEV replicon RNA, capsid protein RNA, and E protein RNA. These RNAs have a low probability of recombination in cells, and may produce VEEV viruses. In order to minimize the recombination risk in the VRP delivery system, a three-plasmid system is usually employed to transcribe VEEV replicon RNA, capsid protein RNA, and E protein RNA, respectively. Even so, the possibility of generating VEEV viruses through recombination cannot be completely avoided. In addition, the VRP delivery system also has some other defects: due to the presence of capsid proteins, on one hand, it limits the exposure of saRNA, and on the other hand, it enhances the host's antiviral immune response, i.e., it not only affects the RNA delivery efficiency but also affects the side reactions during the drug use. Therefore, the VRP delivery system is usually only used for gene delivery in a few fields such as vaccines. For the above reasons, the entire biomedical field is striving to develop new and more potential gene delivery systems, but it is full of challenges to accurately and effectively deliver these molecules into cells while ensuring safety.

Viral E proteins can be assembled relying on negatively charged region and the signal peptide at their C-terminus to form the envelope structure of viral particles. During the viral life cycle, viral E proteins interact with other viral components, such as nucleoproteins, matrix proteins, etc., to package infectious viral particles. The process of viral replication and packaging to produce progeny viruses is completely dependent on the presence of viral structural proteins such as viral nucleoproteins, matrix proteins, etc. When the viral genome lacks structural genes such as nucleoproteins, matrix proteins, etc., it usually cannot produce progeny viral particles.

The interaction between RNA and proteins is one of the decisive factors for the realization of cellular physiological processes. In recent years, with the improvement of technologies and the establishment of new methods, significant progress has been made in the research on the interaction between RNA and proteins. At present, researchers have identified many protein-binding sites on RNA, discovered many RNA-binding domains in proteins, and conducted relatively detailed studies on their structural characteristics. Viral E protein is the main membrane protein on the viral envelope, which not only mediates the binding of the virus to cell receptors but also plays an important role in the formation of the viral envelope.

Therefore, how to explore the potential of related interaction between viral E proteins and mRNA, develop a new mRNA drug delivery system by utilizing the characteristics of viral E proteins in combination with the safety and efficiency of saRNA, and develop new and more mature mRNA drug preparations require further exploration by those skilled in the art.

### CONTENT OF THE INVENTION

To address the issues existing in the prior art, the present disclosure aims to provide a novel saRNA delivery (VRV) system and construction method therefor. By introducing saRNA sequences and viral envelope protein RNA sequences into cells via a two-plasmid transfection system, a cellular microvesicle delivery system containing saRNA can be effectively packaged, which is used for packaging and delivering saRNA, and achieving the expression of target proteins through saRNA. This new form of delivery system greatly expands the ways of gene drug delivery.

To achieve the above purposes, the technical solutions of the present disclosure are as follows:
In one aspect, the present disclosure provides a method for constructing an saRNA microvesicle delivery system, including the following steps:
(1) designing an saRNA expression vector 1 and a membrane protein expression vector 2;
(2) co-transfecting the expression vector 1 and the expression vector 2 into a host cell;
(3) culturing the host cell and isolating to obtain a microvesicle containing saRNA;

wherein, the saRNA comprises a target gene sequence;
the replicon in the saRNA expression vector is an alphavirus replicon;
the microvesicle does not comprise a capsid protein.

In another aspect, the present disclosure provides a microvesicle delivery vector prepared by the aforementioned method, wherein the delivery vector comprises a microvesicle with a viral membrane protein embedded on the surface and saRNA packaged in the vesicle, the microvesicle does not comprise a capsid protein; preferably, the saRNA also comprises a target gene sequence.

In another aspect, the present disclosure provides a cell stably expressing the aforementioned microvesicle delivery vector.

In another aspect, the present disclosure provides a use of the aforementioned method, microvesicle delivery vector and/or cell in the manufacture of a targeted drug.

In another aspect, the present disclosure provides a use of the aforementioned method, microvesicle delivery vector and/or cell in the manufacture of a vaccine preparation.

### The beneficial effects achieved by the present disclosure are at least as follows:

Through research on the interaction between saRNA and viral membrane proteins, the present disclosure has found that viral E proteins can interact with saRNA, and in mammalian cells, viral E proteins can efficiently mediate the production of cellular microvesicles containing saRNA. This interaction may depend on the specific transmembrane domain and protein's tertiary structure in the intracellular domain of the viral E protein, as well as the tertiary structure of saRNA. When viral membrane proteins are absent, although cells can also spontaneously produce extracellular vesicles, they cannot effectively package saRNA into the vesicles; at the same time, there are certain differences in the interaction effects between different viral E proteins and saRNA, and the efficiency of producing microvesicles packaging saRNA also varies. Optimizing the viral E protein RNA sequence and saRNA sequence can effectively promote the interaction between viral E proteins and saRNA, thereby significantly improving the production efficiency of microvesicles packaging saRNA.

The present disclosure enable the effective packaging of cellular microvesicles containing saRNA by introducing saRNA sequences and viral envelope protein RNA sequences into cells via a two-plasmid transfection system. This novel saRNA delivery (VRV) system mediated by viral envelope protein-assembled microvesicles can effectively mediate *in vivo* delivery of saRNA and has outstanding delivery advantages. The VRV delivery system differs from VLV in that it is not an active virus. Only the saRNA drug component is delivered into the body, without any viral structural protein genes, and it cannot produce progeny viruses. This overcomes the fatal drawbacks of viral vectors and ensures favorable safety in use. Secondly, the VRV delivery system is different from VRP: VRP is a virus-like particle of alphavirus, whose key mechanism of saRNA packaging lies in the interaction between saRNA and nucleoproteins. Therefore, viral nucleoprotein is crucial for the formation of VRP, and VRP comprises a large amount of viral nucleoprotein components; VRV is a type of cellular microvesicles, whose key mechanism of saRNA packaging is the interaction between saRNA and viral membrane proteins. VRV does not comprise viral nucleoprotein components and has lower immunogenicity, making it more suitable for RNA delivery in medical fields such as protein replacement drugs, antibody drugs, cell and gene therapy drugs, etc. In addition, the VRV packaging system does not require the use of viral nucleoprotein RNA. The packaging of VRV can be completed using a two-plasmid system, while avoiding the translation of viral nucleoproteins, which is more conducive to the cell-specific translation of viral membrane proteins. Thus, the VRV system has better packaging efficiency. Furthermore, since the viral membrane proteins can bind to corresponding cell receptors, and the expression of cell receptors varies across different tissues, the VRV delivery systems constructed using different viral membrane proteins can achieve targeting of different tissues, thereby endowing the VRV system with tissue-targeted delivery functionality. Therefore, the VRV delivery system constructed in the present disclosure can be modified with different elements to develop a modular platform with high efficiency and low immunogenicity for different diseases and different targets. By efficiently delivering saRNA into cells or the body, the efficient and sustained expression of the carried target proteins is achieved.

### DESCRIPTION OF THE DRAWINGS

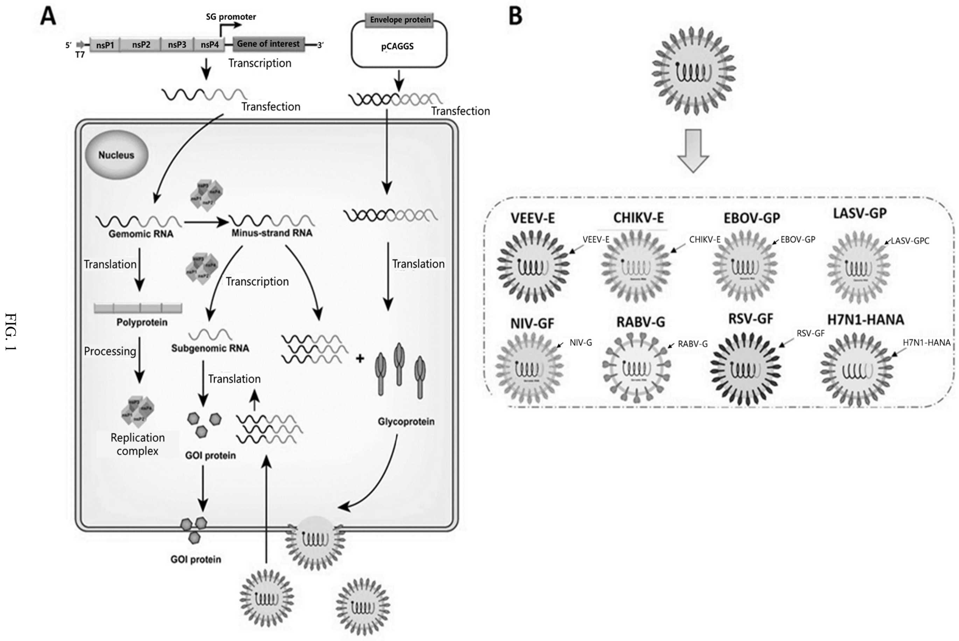

FIG. 1 shows a schematic diagram of the construction of a novel saRNA delivery (VRV) system mediated by viral envelope protein-assembled microvesicles and a schematic diagram of microvesicles of different viral envelopes obtained from this VRV system, wherein FIG. 1A: a schematic diagram of microvesicles formed after co-transfection of saRNA and viral envelope protein expression plasmids and their delivery into cells to express target proteins; FIG. 1B: microvesicles formed by different viral envelope proteins constructed by this VRV platform.

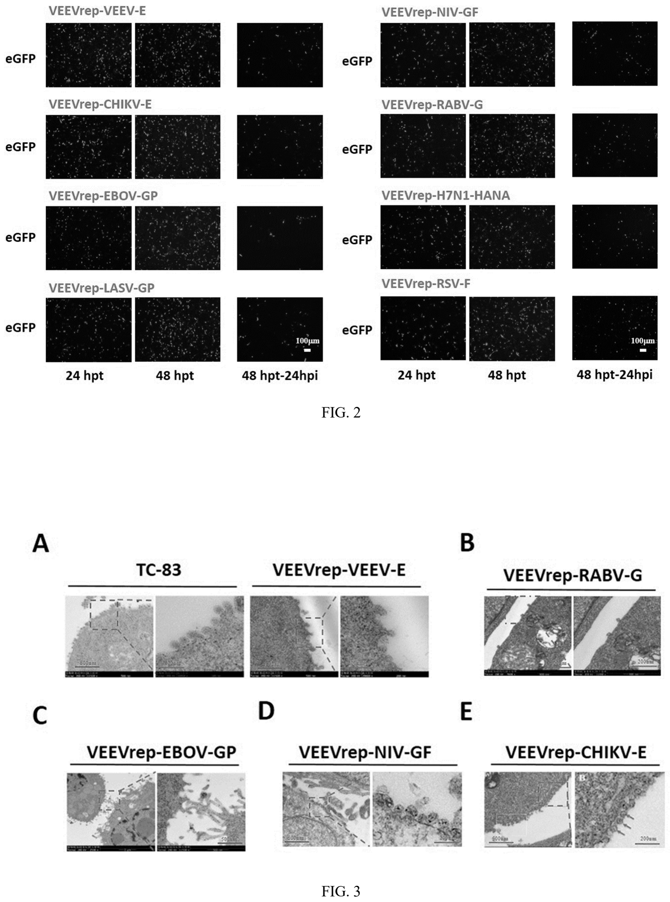

FIG. 2 shows the packaging efficiency and the expression of target proteins of different viral envelope proteins in the VRV system in naïve BHK-21 cells, including CHIKV-E, VEEV-E, EBOV-GP, NIV-GF, LASV-GP, RABV-G, H7N1-HANA, RSV-F, where the expression of the target proteins is indicated by the fluorescent protein EGFP.
FIG. 3 shows the electron microscopic morphology of microvesicles of different viral envelopes constructed by the VRV system, wherein FIG. 3A: a comparison of the electron microscopic structure of the VEEV virus TC-83 strain and the electron microscopic structure of VEEVrep-VEEV-E microvesicles assembled by SaRNA-VEEV envelope glycoprotein E; FIG. 3B: the electron microscopic structure of VEEVrep-RABV-G microvesicles assembled by VEEV replicon-rabies virus envelope glycoprotein G; FIG. 3C: the electron microscopic structure of VEEVrep-EBOV-GP microvesicles assembled by VEEV replicon-Ebola virus envelope glycoprotein G; FIG. 3D: the electron microscopic structure of VEEVrep-NIV-GF microvesicles assembled by VEEV replicon-Nipah virus envelope glycoprotein GF; FIG. 3E: the electron microscopic structure of VEEVrep-CHIKV-E microvesicles assembled by VEEV replicon-Chikungunya virus envelope glycoprotein E. Bar: 500 nm and 200 nm.

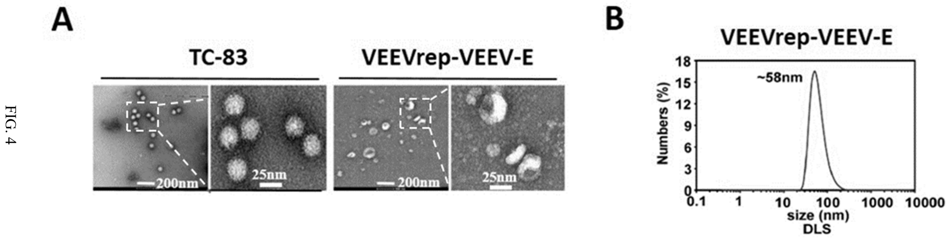

FIG. 4 shows the negative staining electron microscopic structure and particle size of VEEVrep-VEEV-E microvesicle membranes obtained via the VRV system, wherein FIG. 4A: a comparison of the electron microscopic structure of the VEEV virus TC-83 strain and the negative staining electron microscopic structure of VEEVrep-VEEV-E microvesicles; FIG. 4B: the particle size of VEEVrep-VEEV-E microvesicles detected by DLS. Bar: 200 nm and 25 nm.

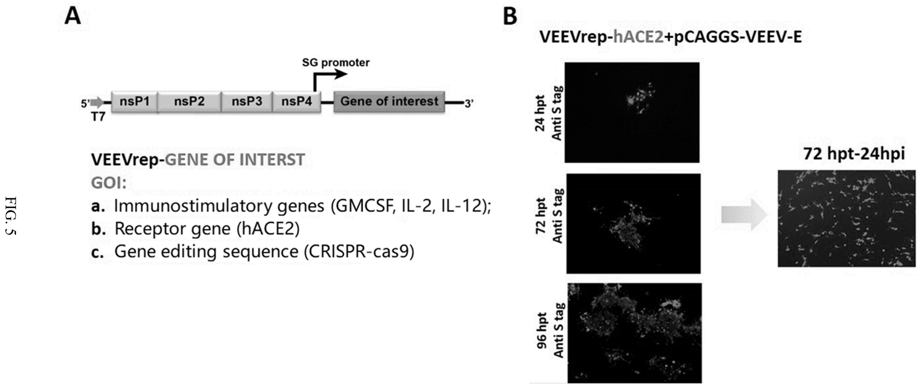

FIG. 5 shows the strategy for delivering target proteins using the VRV system and the delivery of hACE2 (human angiotensin-converting enzyme II) achieved by this strategy, wherein FIG. 5A: a schematic diagram of introducing target proteins after saRNA and the types of optional target proteins; FIG. 5B: a delivery of hACE2 loaded in saRNA via the VRV system and the verification of the hACE2 expression after transfection with microvesicles.

### SPECIFIC IMPLEMENTATIONS

In the following examples of the present disclosure, the experimental methods without specifying specific conditions are generally carried out under conventional conditions or the conditions recommended by the manufacturers. Various commonly used chemical and biological reagents used in the examples are commercially available products.

Unless otherwise defined, all technical and scientific terms used in the present disclosure have the same meanings as commonly understood by those skilled in the technical field to which the present disclosure belongs. The terms used in the description of the present disclosure are only for the purpose of describing specific examples and are not intended to limit the present disclosure.

The terms "comprising" and "having" and any variations thereof used herein are intended to cover non-exclusive inclusions. For example, a process, method, device, product or equipment that includes a series of steps is not limited to the steps or modules listed, but optionally further includes unlisted steps, or optionally further includes other steps inherent to the process, method, product, or equipment.

The term "more" mentioned herein refers to two or more. "And/or" describes the association relationship of associated objects, indicating that there can be three types of relationships, for example, A and/or B can represent three scenarios: A exists alone, A and B exist simultaneously, and B exists alone. The character "/" generally refers to that the associated objects before and after are in an "or" relationship. Meanwhile, for a better understanding of the present disclosure, definitions and explanations of relevant terms are provided below.

The term "vector" used herein refers to a nucleic acid carrier tool into which polynucleotides can be inserted. When a vector enables the expression of a protein encoded by the inserted polynucleotide, the vector is called an expression vector. Vectors can be introduced into host cells by transformation, transduction, or transfection, enabling the genetic material elements they carry to be expressed in the host cells. Vectors are well-known to those skilled in the art, including but not limited to: plasmids; phagemids; cosmids; artificial chromosomes, such as yeast artificial chromosomes (YACs), bacterial artificial chromosomes (BACs) or P1-derived artificial chromosomes (PACs); phages such as λ phages or M13 phages, and animal viruses, etc. Animal viruses that can be used as vectors include, but are not limited to, retroviruses (including lentiviruses), adenoviruses, adeno-associated viruses, herpes viruses (such as herpes simplex virus), poxviruses, baculoviruses, papillomaviruses, and papovaviruses (such as SV40). A vector may contain multiple elements that control expression, including but not limited to promoter sequences, transcription initiation sequences, enhancer sequences, selection elements and reporter genes. In addition, the vector may also contain an origin of replication.

The term "host cell" used herein refers to a recipient cell that accepts exogenous genes during transformation and transduction (infection), including but not limited to, prokaryotic cells such as *Escherichia coli* or *Bacillus subtilis,* fungal cells such as yeast cells or *Aspergillus,* insect cells such as Drosophila S2 cells or Sf9 cells, or animal cells such as fibroblasts, CHO cells, COS cells, NSO cells, HeLa cells, BHK cells, HEK 293 cells or human cells, etc.

The term "microvesicles" used herein refers to extracellular vehicles (EVs) formed by direct cell budding outward or "extrusion" from cells, containing cell membranes and partial cytoplasmic components. The particle size of EVs ranges from 30 nm~1000 nm, and the surface contains proteins such as CD40, integrins, selectins, etc. The microvesicles described herein are a type of EVs comprising viral envelope proteins, with an average particle size of about58 nm.

The term "saRNA" used herein, i.e., self-amplifying RNA, is a short single-stranded oligonucleotide that can self-replicate using its own RNA sequence as a template. In addition, saRNA also carries a sequence capable of expressing RNA polymerase (RNA-dependent RNA polymerase, RdRP).

The term "core region" used herein refers to the capsid core region formed by viral capsid proteins (CAs). Taking mature and infectious HIV-1 as an example, the HIV-1 viral genome is located within a conical capsid core composed of viral capsid proteins. The capsid core is assembled from approximately 250 CA hexamers and 12 pentamers. The CA subunit is further composed of two structural domains, namely the N-terminal domain (NTD) and the C-terminal domain (CTD).

In one aspect, the present disclosure provides a use of alphavirus replicons and viral envelope proteins in constructing a saRNA delivery system. The saRNA delivery system is a microvesicle encapsulating saRNA, and the saRNA comprises a target gene sequence. The microvesicle is a vesicular body with a bilayer membrane structure assembled from a viral envelope protein and a lipid bilayer, not comprising a capsid protein.

In one aspect, the present disclosure provides a method for constructing an saRNA microvesicle delivery system, including the following steps:
(1) designing an saRNA expression vector 1 and a membrane protein expression vector 2;
(2) co-transfecting the expression vector 1 and the expression vector 2 into a host cell;
(3) culturing the host cell and isolating to obtain a microvesicle containing saRNA;

wherein the saRNA comprises a target gene sequence;
the replicon in the saRNA expression vector is an alphavirus replicon;
the microvesicle does not comprise a capsid protein.

In some embodiments, the nucleotide sequence of the alphavirus replicon is as shown in SEQ ID NO.3.

In some embodiments, the expression vector comprises plasmids, phages and viruses.

In some embodiments, the expression vector of the saRNA comprising the target gene sequence is a plasmid.

In some embodiments, the expression vector of the gene sequence of the viral envelope protein is a plasmid.

In some embodiments, the membrane protein is a viral membrane protein.

In some embodiments, the viral membrane protein is selected from the viruses of *Alphavirus, Ebola virus, Arenavirus, Henipavirus, Lyssavirus, Pneumovirus, Alpharetrovirus, Lentivirus, Vesiculovirus, Marburgvirus, Mumpsvirus, Morbillivirus, Influenzavirus A, Influenzavirus B, Influenzavirus C, Hantavirus, Flavivirus, Hepacivirus,* and/or *Rubivirus.*

In some embodiments, the viral membrane protein is selected from CHIKV, VEEV, EBOV, NIV, LASV, RABV, RSV and/or H7N1 viruses.

In some embodiments, the target gene is a therapeutic gene or an antigen gene.

In some embodiments, the therapeutic gene is an immunostimulatory gene or a suicide gene.

In some embodiments, the immunostimulatory gene encodes an immunostimulatory protein;
In some embodiments, the immunostimulatory protein is selected from cytokines, chemokines, interferons, tumor necrosis factors (TNF), colony-stimulating factors, proteins exposed to APCs (antigen-presenting cells), growth factors, Class I or Class II major histocompatibility complex (MHC) components, inducers or inhibitors of apoptosis, cell growth inhibitors, immunotoxins, and blockers of immune escape mechanisms of immune checkpoint inhibitors;
In some embodiments, the cytokine is an interleukin; more preferably, the cytokine is selected from IL-2, IL-6, IL-12, IL-15, and IL-24;
In some embodiments, the chemokine is selected from CXCL10, CXCL9, and CXCL11;
In some embodiments, the interferon is selected from IFNγ and IFNα;
In some embodiments, the colony-stimulating factor is selected from GM-CSF, G-CSF, and M-CSF;
In some embodiments, the protein of the APC (antigen-presenting cell) is selected from B7.1 and B7.2;
In some embodiments, the growth factor is selected from transforming growth factor TGF, fibroblast growth factor FGF, and vascular endothelial growth factor VEGF;
In some embodiments, the inducer or inhibitor of apoptosis is selected from one or more of Bax, Bcl2, and/or BclX;
In some embodiments, the cell growth inhibitor is selected from p21, p16, and Rb;
In some embodiments, the suicide gene is selected from thymidine kinase (TK), thymidylate kinase, cytosine deaminase (CDase), and uracil phosphoribosyltransferase (UPRTase);
In some embodiments, the antigen gene is a receptor gene, such as hACE2;
In some embodiments, the nucleotide sequence of hACE2 is as shown in SEQ ID NO.15.

In another aspect, the present disclosure provides a microvesicle delivery vector prepared by the aforementioned method, wherein the delivery vector comprises a microvesicle with a viral membrane protein embedded on the surface and saRNA packaged in the vesicle, the microvesicle does not comprise a capsid protein.

In some embodiments, the saRNA in the microvesicle delivery vector further comprises a target gene sequence.

In some embodiments, the microvesicle is a vesicular body with a bilayer membrane structure assembled from a viral envelope protein and a lipid bilayer.

In some embodiments, the particle size of the microvesicle is about 10 nm-1000 nm.

In some embodiments, the particle size of the microvesicle is about 50-100 nm.

In some embodiments, the particle size of the microvesicle is about 58 nm.

In another aspect, the present disclosure provides a cell that stably expresses the aforementioned microvesicle delivery vector.

In another aspect, the present disclosure provides a use of the aforementioned method, microvesicle delivery vector, and/or cell in the manufacture of a targeted drug.

In another aspect, the present disclosure provides a use of the aforementioned method, microvesicle delivery vector, and/or cell in the manufacture of a vaccine preparation.

### Examples

The technical solutions of the present disclosure will be further described below through specific embodiments. Those skilled in the art should understand that the examples are only for helping understand the present disclosure and should not be regarded as specific limitations on the present disclosure.

The experimental methods involved in this section, such as PCR, enzyme digestion, ligation, transformation, RNA extraction, and RT-PCR, etc., unless otherwise specified, all adopt conventional methods in the field. The following enumerated are only several specific examples of the present disclosure. Obviously, the present disclosure is not limited to the following examples, and there can be many variations. Therefore, modifications or improvements made by those skilled in the art on the basis of the content of the present disclosure shall all fall within the scope of protection claimed by the present disclosure.

### Example 1: Construction of a Novel SaRNA Delivery System Mediated by Viral Envelope Protein-Assembled Microvesicles

During the exploration of the present disclosure, it was found that the RNA fragments of alphavirus replicons in host cells can be mediated to be packaged by the envelope proteins of various viruses to form cellular microvesicles. Therefore, the present disclosure utilizes the assembly ability of viral envelope proteins and the self-replication ability of the alphavirus genome, after modification and design, constructs a novel saRNA system delivery platform mediated by viral envelope protein-assembled microvesicles (Virus-Replicon vesicles, VRV) for packaging and delivering saRNA, and realizes the expression of downstream target proteins through saRNA.

The specific construction schematic diagram is shown in FIG. 1. The saRNA-GOI plasmid expressing the target protein (Gene of Interest, GOI) is constructed by reverse genetics technology, and saRNA-GOI RNA is obtained through *in vitro* transcription. The saRNA-GOI RNA and the eukaryotic expression plasmid expressing the viral envelope protein are co-transcribed in host cells. After entering the cell, the saRNA-GOI RNA is translated into a replication complex. Under the action of the replication complex, the progeny RNA and the target protein are expressed; at the same time, the envelope protein expression plasmid entering the host cell expresses the envelope proteins. The envelope proteins self-assemble near the lipid membrane to form microvesicles encapsulating saRNA. In the present disclosure, microvesicles assembled by different viral envelope proteins are constructed through this VRV system construction method, including CHIKV, VEEV, EBOV, NIV, LASV, RABV, H7N1, and RSV (see FIG. 1B).

### Example 2: Packaging Efficiency and Expression of Target Proteins of Different Viral Envelope Proteins in the VRV System

According to the strategy in Example 1, the VEEV replicon RNA (nsp1-nsp4) expressing EGFP plasmid (pacyc-VEEVrep-EGFP) was firstly constructed in this Example, whose nucleotide sequence is shown in SEQ ID NO.1, wherein the vector sequence is shown in SEQ ID NO.2, the VEEVrep sequence is shown in SEQ ID NO.3, and the EGFP sequence is shown in SEQ ID NO.4. The construction method is as follows: using the full-length infectious clone of VEEV vaccine strain pacyc-TC-83 (preserved in our laboratory) as a template, the structural protein region sequence in TC-83 was replaced with the EGFP sequence by using AscI and PacI restriction enzyme sites to obtain the pacyc-VEEVrep-EGFP clone. The pacyc-VEEVrep-EGFP was linearized through the NotI restriction enzyme site. VEEVrep-EGFP saRNA was obtained by *in vitro* transcription of RNA using the T7 mMESSAGE mMACHINE kit (TAKARA).

The nucleotide sequence of SEQ ID NO.1 is as follows:

ATGggcggcgcatgagagaagcccagaccaattacctacccaaaatggagaaagttcacgttgacatcgaggaagacagcccattcc
tcagagctttgcagcggagcttcccgcagtttgaggtagaagccaagcaggtcactgataatgaccatgctaatgccagagcgttttcgcatctgg
cttcaaaactgatcgaaacggaggtggacccatccgacacgatccttgacattggaagtgcgcccgcccgcagaatgtattctaagcacaagtat
cattgtatctgtccgatgagatgtgcggaagatccggacagattgtataagtatgcaactaagctgaagaaaaactgtaaggaaataactgataag
gaattggacaagaaaatgaaggagctcgccgccgtcatgagcgaccctgacctggaaactgagactatgtgcctccacgacgacgagtcgtgt
cgctacgaagggcaagtcgctgtttaccaggatgtatacgcggttgacggaccgacaagtctctatcaccaagccaataagggagttagagtcg
cctactggataggctttgacaccaccccttttatgtttaagaacttggctggagcatatccatcatactctaccaactgggccgacgaaaccgtgtta
acggctcgtaacataggcctatgcagctctgacgttatggagcggtcacgtagagggatgtccattcttagaaagaagtatttgaaaccatccaac
aatgttctattctctgttggctcgaccatctaccacgagaagagggacttactgaggagctggcacctgccgtctgtatttcacttacgtggcaagca
aaattacacatgtcggtgtgagactatagttagttgcgacgggtacgtcgttaaaagaatagctatcagtccaggcctgtatgggaagccttcaggc
tatgctgctacgatgcaccgcgagggattcttgtgctgcaaagtgacagacacattgaacggggagagggtctcttttcccgtgtgcacgtatgtg
ccagctacattgtgtgaccaaatgactggcatactggcaacagatgtcagtgcggacgacgcgcaaaaactgctggttgggctcaaccagcgtat
agtcgtcaacggtcgcacccagagaaacaccaataccatgaaaaattaccttttgcccgtagtggcccaggcatttgctaggtgggcaaaggaat
ataaggaagatcaagaagatgaaaggccactaggactacgagatagacagttagtcatggggtgttgttgggcttttagaaggcacaagataaca
tctatttataagcgcccggatacccaaaccatcatcaaagtgaacagcgatttccactcattcgtgctgcccaggataggcagtaacacattggaga
tcgggctgagaacaagaatcaggaaaatgttagaggagcacaaggagccgtcacctctcattaccgccgaggacgtacaagaagctaagtgcg
cagccgatgaggctaaggaggtgcgtgaagccgaggagttgcgcgcagctctaccacctttggcagctgatgttgaggagcccactctggaag
ccgatgtcgacttgatgttacaagaggctggggccggctcagtggagacacctcgtggcttgataaaggttaccagctacgctggcgaggacaa
gatcggctcttacgctgtgctttctccgcaggctgtactcaagagtgaaaaattatcttgcatccaccctctcgctgaacaagtcatagtgataacac
actctggccgaaaagggcgttatgccgtggaaccataccatggtaaagtagtggtgccagagggacatgcaatacccgtccaggactttcaagc
tctgagtgaaagtgccaccattgtgtacaacgaacgtgagttcgtaaacaggtacctgcaccatattgccacacatggaggagcgctgaacactg

atgaagaatattacaaaactgtcaagcccagcgagcacgacggcgaatacctgtacgacatcgacaggaaacagtgcgtcaagaaagaactag
tcactgggctagggctcacaggcgagctggtggatcctcccttccatgaattcgcctacgagagtctgagaacacgaccagccgctccttaccaa
gtaccaaccataggggtgtatggcgtgccaggatcaggcaagtctggcatcattaaaagcgcagtcaccaaaaaagatctagtggtgagcgcca
agaaagaaaactgtgcagaaattataagggacgtcaagaaaatgaaagggctggacgtcaatgccagaactgtggactcagtgctcttgaatgg
atgcaaacaccccgtagagaccctgtatattgacgaagcttttgcttgtcatgcaggtactctcagagcgctcatagccattataagacctaaaaag
gcagtgctctgcggggatcccaaacagtgcggtttttttaacatgatgtgcctgaaagtgcattttaaccacgagatttgcacacaagtcttccacaa
aagcatctctcgccgttgcactaaatctgtgacttcggtcgtctcaaccttgttttacgacaaaaaaatgagaacgacgaatccgaaagagactaag
attgtgattgacactaccggcagtaccaaacctaagcaggacgatctcattctcacttgtttcagagggtgggtgaagcagttgcaaatagattaca
aaggcaacgaaataatgacggcagctgcctctcaagggctgacccgtaaaggtgtgtatgccgttcggtacaaggtgaatgaaaatcctctgtac
gcacccacctcagaacatgtgaacgtcctactgacccgcacggaggaccgcatcgtgtggaaaacactagccggcgacccatggataaaaac
actgactgccaagtaccctgggaatttcactgccacgatagaggagtggcaagcagagcatgatgccatcatgaggcacatcttggagagaccg
gaccctaccgacgtcttccagaataaggcaaacgtgtgttgggccaaggctttagtgccggtgctgaagaccgctggcatagacatgaccactg
aacaatggaacactgtggattattttgaaacggacaaagctcactcagcagagatagtattgaaccaactatgcgtgaggttctttggactcgatctg
gactccggtctattttctgcacccactgttccgttatccattaggaataatcactgggataactccccgtcgcctaacatgtacgggctgaataaaga
agtggtccgtcagctctctcgcaggtacccacaactgcctcgggcagttgccactggaagagtctatgacatgaacactggtacactgcgcaatta
tgatccgcgcataaacctagtacctgtaaacagaagactgcctcatgctttagtcctccaccataatgaacacccacagagtgacttttcttcattcgt
cagcaaattgaagggcagaactgtcctggtggtcggggaaaagttgtccgtcccaggcaaaatggttgactggttgtcagaccggcctgaggct
accttcagagctcggctggatttaggcatcccaggtgatgtgcccaaatatgacataatatttgttaatgtgaggaccccatataaataccatcactat
cagcagtgtgaagaccatgccattaagcttagcatgttgaccaagaaagcttgtctgcatctgaatcccggcggaacctgtgtcagcataggttatg
gttacgctgacagggccagcgaaagcatcattggtgctatagcgcggcagttcaagttttcccgggtatgcaaaccgaaatcctcacttgaagag
acggaagttctgtttgtattcattgggtacgatcgcaaggcccgtacgcacaatccttacaagctttcatcaaccttgaccaacatttatacaggttcc
agactccacgaagccggatgtgcaccctcatatcatgtggtgcgaggggatattgccacggccaccgaaggagtgattataaatgctgctaaca
gcaaaggacaacctggcggaggggtgtgcggagcgctgtataagaagttcccggaaagcttcgatttacagccgatcgaagtaggaaaagcg
cgactggtcaaaggtgcagctaaacatatcattcatgccgtaggaccaaacttcaacaaagtttcggaggttgaaggtgacaaacagttggcaga
ggcttatgagtccatcgctaagattgtcaacgataacaattacaagtcagtagcgattccactgttgtccaccggcatcttttccgggaacaaagatc
gactaacccaatcattgaaccatttgctgacagctttagacaccactgatgcagatgtagccatatactgcagggacaagaaatgggaaatgactc
tcaaggaagcagtggctaggagagaagcagtggaggagatatgcatatccgacgactcttcagtgacagaacctgatgcagagctggtgaggg
tgcatccgaagagttctttggctggaaggaagggctacagcacaagcgatggcaaaactttctcatatttggaagggaccaagtttcaccaggcg
gccaaggatatagcagaaattaatgccatgtggcccgttgcaacggaggccaatgagcaggtatgcatgtatatcctcggagaaagcatgagca
gtattaggtcgaaatgccccgtcgaagagtcggaagcctcctcaccacctagcacgctgccttgcttgtgcatccatgccatgactccagaaaga
gtacagcgcctaaaagcctcacgtccagaacaaattactgtgtgctcatcctttccattgccgaagtatagaatcactggtgtgcagaagatccaat
gctcccagcctatattgttctcaccgaaagtgcctgcgtatattcatccaaggaagtatctcgtggaaacaccaccggtagacgagactccggagc
catcggcagagaaccaatccacagaggggacacctgaacaaccaccacttataaccgaggatgagaccaggactagaacgcctgagccgatc
atcatcgaagaggaagaagaggatagcataagtttgctgtcagatggcccgacccaccaggtgctgcaagtcgaggcagacattcacgggccg

cccctctgtatctagctcatcctggtccattcctcatgcatccgactttgatgtggacagtttatccatacttgacaccctggagggagctagcgtgacc
agcggggcaacgtcagccgagactaactcttacttcgcaaagagtatggagtttctggcgcgaccggtgcctgcgcctcgaacagtattcagga
accctccacatcccgctccgcgcacaagaacaccgtcacttgcacccagcagggcctgctcgagaaccagcctagtttccaccccgccaggcg
tgaatagggtgatcactagagaggagctcgaggcgcttaccccgtcacgcactcctagcaggtcggtctcgagaaccagcctggtctccaaccc
gccaggcgtaaatagggtgattacaagagaggagtttgaggcgttcgtagcacaacaacaatgacggtttgatgcgggtgcatacatcttttcctc
cgacaccggtcaagggcatttacaacaaaaatcagtaaggcaaacggtgctatccgaagtggtgttggagaggaccgaattggagatttcgtatg
ccccgcgcctcgaccaagaaaaagaagaattactacgcaagaaattacagttaaatcccacacctgctaacagaagcagataccagtccaggaa
ggtggagaacatgaaagccataacagctagacgtattctgcaaggcctagggcattatttgaaggcagaaggaaaagtggagtgctaccgaacc
ctgcatcctgttcctttgtattcatctagtgtgaaccgtgccttctcaagccccaaggtcgcagtggaagcctgtaacgccatgttgaaagagaacttt
ccgactgtggcttcttactgtattattccagagtacgatgcctatttggacatggttgacggagcttcatgctgcttagacactgccagtttttgccctg
caaagctgcgcagctttccaaagaaacactcctatttggaacccacaatacgatcggcagtgccttcagcgatccagaacacgctccagaacgtc
ctggcagctgccacaaaaagaaattgcaatgtcacgcaaatgagagaattgcccgtattggattcggcggcctttaatgtggaatgcttcaagaaa
tatgcgtgtaataatgaatattgggaaacgtttaaagaaaaccccatcaggcttactgaagaaaacgtggtaaattacattaccaaattaaaaggac
caaaagctgctgctctttttgcgaagacacataatttgaatatgttgcaggacataccaatggacaggtttgtaatggacttaaagagagacgtgaaa
gtgactccaggaacaaaacatactgaagaacggcccaaggtacaggtgatccaggctgccgatccgctagcaacagcgtatctgtgcggaatc
caccgagagctggttaggagattaaatgcggtcctgcttccgaacattcatacactgtttgatatgtcggctgaagactttgacgctattatagccga
gcacttccagcctggggattgtgttctggaaactgacatcgcgtcgtttgataaaagtgaggacgacgccatggctctgaccgcgttaatgattctg
gaagacttaggtgtggacgcagagctgttgacgctgattgaggcggctttcggcgaaatttcatcaatacatttgcccactaaaactaaatttaaatt
cggagccatgatgaaatctggaatgttcctcacactgtttgtgaacacagtcattaacattgtaatcgcaagcagagtgttgagagaacggctaacc
ggatcaccatgtgcagcattcattggagatgacaatatcgtgaaaggagtcaaatcggacaaattaatggcagacaggtgcgccacctggttgaa
tatggaagtcaagattatagatgctgtggtgggcgagaaagcgccctatttctgtggagggtttattttgtgtgactccgtgaccggcacagcgtgc
cgtgtggcagaccccctaaaaaggctgtttaagcttggcaaacctctggcagcagacgatgaacatgatgatgacaggagaagggcattgcatg
aagagtcaacacgctggaaccgagtgggtattctttcagagctgtgcaaggcagtagaatcaaggtatgaaaccgtaggaacttccatcatagtta
tggccatgactactctagctagcagtgttaaatcattcagctacctgagaggggcccctataactctctacggctaacctgaatggactacgacatc
gatggcgcgccACCATGGTGAGCAAGGGCGAGGAGCTGTTCACCGGGGTGGTGCCCATCCTG
GTCGAGCTGGACGGCGACGTAAACGGCCACAAGTTCAGCGTGTCCGGCGAGGGCGAGG
GCGATGCCACCTACGGCAAGCTGACCCTGAAGTTCATCTGCACCACCGGCAAGCTGCCC
GTGCCCTGGCCCACCCTCGTGACCACCCTGACCTACGGCGTGCAGTGCTTCAGCCGCTA
CCCCGACCACATGAAGCAGCACGACTTCTTCAAGTCCGCCATGCCCGAAGGCTACGTCC
AGGAGCGCACCATCTTCTTCAAGGACGACGGCAACTACAAGACCCGCGCCGAGGTGAA
GTTCGAGGGCGACACCCTGGTGAACCGCATCGAGCTGAAGGGCATCGACTTCAAGGAG
GACGGCAACATCCTGGGGCACAAGCTGGAGTACAACTACAACAGCCACAACGTCTATAT
CATGGCCGACAAGCAGAAGAACGGCATCAAGGTGAACTTCAAGATCCGCCACAACATCG
AGGACGGCAGCGTGCAGCTCGCCGACCACTACCAGCAGAACACCCCCATCGGCGACGG

CCCCGTGCTGCTGCCCGACAACCACTACCTGAGCACCCAGTCCGCCCTGAGCAAAGACC
CCAACGAGAAGCGCGATCACATGGTCCTGCTGGAGTTCGTGACCGCCGCCGGGATCACT
CTCGGCATGGACGAGCTGTACAAGGCTAGCATCGAGGGCCGGGGCGGCTGGGGCAAAG
AGACAGCCGCCGCCAAGTTCGAGCGGCAGCACATGGACAGCTGATAAttaattaagtaaccgataca
gcagcaattggcaagctgcttacatagaactcgcggcgattggcatgccgctttaaaatttttattttattttttcttttcttttccgaatcggattttgttttta
atatttcaaaaaaaaaaaaaaaaaaaaaaaaaaaaaaaaaaaaaaaaaaaaaaaaaaggaagagcgcggccgcaTCCGGCGTTCA
GCCTGTGCCACAGCCGACAGGATGGTGACCACCATTTGCCCCATATCACCGTCGGTACTG
ATCCCGTCGTCAATAAACCGAACCGCTACACCCTGAGCATCAAACTCTTTTATCAGTTGG
ATCATGTCGGCGGTGTCGCGGCCAAGACGGTCGAGCTTCTTCACCAGAATGACATCACCT
TCCTCCACCTTCATCCTCAGCAAATCCAGCCCTTCCCGATCTGTTGAACTGCCGGATGCCT
TGTCGGTAAAGATGCGGTTAGCTTTTACCCCTGCATCTTTGAGCGCTGAGGTCTGCCTCG
TGAAGAAGGTGTTGCTGACTCATACCAGGCCTGAATCGCCCCATCATCCAGCCAGAAAG
TGAGGGAGCCACGGTTGATGAGAGCTTTGTTGTAGGTGGACCAGTTGGTGATTTTGAAC
TTTTGCTTTGCCACGGAACGGTCTGCGTTGTCGGGAAGATGCGTGATCTGATCCTTCAAC
TCAGCAAAAGTTCGATTTATTCAACAAAGCCGCCGTCCCGTCAAGTCAGCGTAATGCTCT
GCCAGTGTTACAACCAATTAACCAATTCTGATTAGAAAAACTCATCGAGCATCAAATGAA
ACTGCAATTTATTCATATCAGGATTATCAATACCATATTTTTGAAAAAGCCGTTTCTGTAAT
GAAGGAGAAAACTCACCGAGGCAGTTCCATAGGATGGCAAGATCCTGGTATCGGTCTGC
GATTCCGACTCGTCCAACATCAATACAACCTATTAATTTCCCCTCGTCAAAAATAAGGTTA
TCAAGTGAGAAATCACCATGAGTGACGACTGAATCCGGTGAGAATGGCAAAAGCTTATG
CATTTCTTTCCAGACTTGTTCAACAGGCCAGCCATTACGCTCGTCATCAAAATCACTCGCA
TCAACCAAACCGTTATTCATTCGTGATTGCGCCTGAGCGAGACGAAATACGCGATCGCTG
TTAAAAGGACAATTACAAACAGGAATCGAATGCAACCGGCGCAGGAACACTGCCAGCG
CATCAACAATATTTTCACCTGAATCAGGATATTCTTCTAATACCTGGAATGCTGTTTTCCCG
GGGATCGCAGTGGTGAGTAACCATGCATCATCAGGAGTACGGATAAAATGCTTGATGGTC
GGAAGAGGCATAAATTCCGTCAGCCAGTTTAGTCTGACCATCTCATCTGTAACATCATTGG
CAACGCTACCTTTGCCATGTTTCAGAAACAACTCTGGCGCATCGGGCTTCCCATACAATC
GATAGATTGTCGCACCTGATTGCCCGACATTATCGCGAGCCCATTTATACCCATATAAATCA
GCATCCATGTTGGAATTTAATCGCGGCCTCGAGCAAGACGTTTCCCGTTGAATATGGCTCA
TAACACCCCTTGTATTACTGTTTATGTAAGCAGACAGTTTTATTGTTCATGATGATATATTTT
TATCTTGTGCAATGTAACATCAGAGATTTTGAGACACAACGTGGCTTTGTTGAATAAATCG
AACTTTTGCTGAGTTGAAGGATCAGATCACGCATCTTCCCGACAACGCAGACCGTTCCGT
GGCAAAGCAAAAGTTCAAAATCACCAACTGGTCCACCTACAACAAAGCTCTCATCAACC
GTGGCTCCCTCACTTTCTGGCTGGATGATGGGGCGATTCAGGCCTGGTATGAGTCAGCAA

CACCTTCTTCACGAGGCAGACCTCAGCGCTAGCGGAGTGTATACTGGCTTACTATGTTGG
CACTGATGAGGGTGTCAGTGAAGTGCTTCATGTGGCAGGAGAAAAAAGGCTGCACCGG
TGCGTCAGCAGAATATGTGATACAGGATATATTCCGCTTCCTCGCTCACTGACTCGCTACG
CTCGGTCGTTCGACTGCGGCGAGCGGAAATGGCTTACGAACGGGGCGGAGATTTCCTGG
AAGATGCCAGGAAGATACTTAACAGGGAAGTGAGAGGGCCGCGGCAAAGCCGTTTTTCC
ATAGGCTCCGCCCCCCTGACAAGCATCACGAAATCTGACGCTCAAATCAGTGGTGGCGA
AACCCGACAGGACTATAAAGATACCAGGCGTTTCCCCCTGGCGGCTCCCTCGTGCGCTCT
CCTGTTCCTGCCTTTCGGTTTACCGGTGTCATTCCGCTGTTATGGCCGCGTTTGTCTCATTC
CACGCCTGACACTCAGTTCCGGGTAGGCAGTTCGCTCCAAGCTGGACTGTATGCACGAA
CCCCCCGTTCAGTCCGACCGCTGCGCCTTATCCGGTAACTATCGTCTTGAGTCCAACCCG
GAAAGACATGCAAAAGCACCACTGGCAGCAGCCACTGGTAATTGATTTAGAGGAGTTAG
TCTTGAAGTCATGCGCCGGTTAAGGCTAAACTGAAAGGACAAGTTTTGGTGACTGCGCT
CCTCCAAGCCAGTTACCTCGGTTCAAAGAGTTGGTAGCTCAGAGAACCTTCGAAAAACC
GCCCTGCAAGGCGGTTTTTTCGTTTTCAGAGCAAGAGATTACGCGCAGACCAAAACGAT
CTCAAGAAGATCATCTTATTAAGGGGTCTGACGCTCAGTGGAACGAAAACTCACGTTAA
GGGATTTTGGTCATGAGATTATCAAAAAGGATCTTCACCTAGATCCTTTTAAATTAAAAAT
GAAGTTTTAAATCAATCTAAAGTATATATGAGTAAACTTGGTCTGACAGTTACCAATGCTT
AATCAGTGAGGCACCTATCTCAGCGATCTGTCTATTTCGTTCATCCATAGTTGCCTGACTC
CCCGTCGTGTAGATAACTACGATACGGGAGGGCTTACCATCTGGCCCCAGTGCTGCAATG
ATACCGCGAGACCCACGCTCACCGGCTCCAGATTTATCAGCAATAAACCAGCCAGCCGG
AAGGGCCGAGCGCAGAAGTGGTCCTGCAACTTTATCCGCCTCCATCCAGTCTATTAATTG
TTGCCGGGAAGCTAGAGTAAGTAGTTCGCCAGTTAATAGTTTGCGCAACGTTGTTGCCAT
TGCTGCAGGCATCGTGGTGTCACGCTCGTCGTTTGGTATGGCTTCATTCAGCTCCGGTTCC
CAACGATCAAGGCGAGTTACATGATCCCCCATGTTGTGCAAAAAAGCGGTTAGCTCCTTC
GGTCCTCCGATCGTTGTCAGAAGTAAGTTGGCCGCAGTGTTATCACTCATGGTTATGGCA
GCACTGCATAATTCTCTTACTGTCATGCCATCCGTAAGATGCTTTTCTGTGACTGGTGAGT
ACTCAACCAAGTCATTCTGAGAATAGTGTATGCGGCGACCGAGTTGCTCTTGCCCGGCGT
CAACACGGGATAATACCGCGCCACATAGCAGAACTTTAAAAGTGCTCATCATTGGAAAAC
GTTCTTCGGGGCGAAAACTCTCAAGGATCTTACCGCTGTTGAGATCCAGTTCGATGTAAC
CCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTG
AATACTCATACTCTTCCTTTTTCAATATTATTGAAGCATTTATCAGGGTTATTGTCTCATGAG
CGGATACATATTTGAATGTATTTAGAAAAATAAACAAATAGGGGTTCCGCGCACATTTCCC
CGAAAAGTGCCACCTGACGTCTAAGAAACCATTATTATCATGACATTAACCTATAAAAATA

GGCGTATCACGAGGCCCTTTCGTCTTCAAGAATTtctagaggatccctaatacgactcactatag

The nucleotide sequence of SEQ ID NO.2 is as follows:

ttaattaagtaaccgatacagcagcaattggcaagctgcttacatagaactcgcggcgattggcatgccgctttaaaatttttattttattttttctt
ttcttttccgaatcggattttgtttttaatatttcaaaaaaaaaaaaaaaaaaaaaaaaaaaaaaaaaaaaaaaaaaaaaaaaaaggaagagcgcgg
ccgcaTCCGGCGTTCAGCCTGTGCCACAGCCGACAGGATGGTGACCACCATTTGCCCCATAT
CACCGTCGGTACTGATCCCGTCGTCAATAAACCGAACCGCTACACCCTGAGCATCAAACT
CTTTTATCAGTTGGATCATGTCGGCGGTGTCGCGGCCAAGACGGTCGAGCTTCTTCACCA
GAATGACATCACCTTCCTCCACCTTCATCCTCAGCAAATCCAGCCCTTCCCGATCTGTTGA
ACTGCCGGATGCCTTGTCGGTAAAGATGCGGTTAGCTTTTACCCCTGCATCTTTGAGCGCT
GAGGTCTGCCTCGTGAAGAAGGTGTTGCTGACTCATACCAGGCCTGAATCGCCCCATCAT
CCAGCCAGAAAGTGAGGGAGCCACGGTTGATGAGAGCTTTGTTGTAGGTGGACCAGTTG
GTGATTTTGAACTTTTGCTTTGCCACGGAACGGTCTGCGTTGTCGGGAAGATGCGTGATC
TGATCCTTCAACTCAGCAAAAGTTCGATTTATTCAACAAAGCCGCCGTCCCGTCAAGTCA
GCGTAATGCTCTGCCAGTGTTACAACCAATTAACCAATTCTGATTAGAAAAACTCATCGA
GCATCAAATGAAACTGCAATTTATTCATATCAGGATTATCAATACCATATTTTTGAAAAAGC
CGTTTCTGTAATGAAGGAGAAAACTCACCGAGGCAGTTCCATAGGATGGCAAGATCCTG
GTATCGGTCTGCGATTCCGACTCGTCCAACATCAATACAACCTATTAATTTCCCCTCGTCA
AAAATAAGGTTATCAAGTGAGAAATCACCATGAGTGACGACTGAATCCGGTGAGAATGG
CAAAAGCTTATGCATTTCTTTCCAGACTTGTTCAACAGGCCAGCCATTACGCTCGTCATCA
AAATCACTCGCATCAACCAAACCGTTATTCATTCGTGATTGCGCCTGAGCGAGACGAAAT
ACGCGATCGCTGTTAAAAGGACAATTACAAACAGGAATCGAATGCAACCGGCGCAGGAA
CACTGCCAGCGCATCAACAATATTTTCACCTGAATCAGGATATTCTTCTAATACCTGGAAT
GCTGTTTTCCCGGGGATCGCAGTGGTGAGTAACCATGCATCATCAGGAGTACGGATAAAA
TGCTTGATGGTCGGAAGAGGCATAAATTCCGTCAGCCAGTTTAGTCTGACCATCTCATCT
GTAACATCATTGGCAACGCTACCTTTGCCATGTTTCAGAAACAACTCTGGCGCATCGGGC
TTCCCATACAATCGATAGATTGTCGCACCTGATTGCCCGACATTATCGCGAGCCCATTTATA
CCCATATAAATCAGCATCCATGTTGGAATTTAATCGCGGCCTCGAGCAAGACGTTTCCCGT
TGAATATGGCTCATAACACCCCTTGTATTACTGTTTATGTAAGCAGACAGTTTTATTGTTCA
TGATGATATATTTTTATCTTGTGCAATGTAACATCAGAGATTTTGAGACACAACGTGGCTTT
GTTGAATAAATCGAACTTTTGCTGAGTTGAAGGATCAGATCACGCATCTTCCCGACAACG
CAGACCGTTCCGTGGCAAAGCAAAAGTTCAAAATCACCAACTGGTCCACCTACAACAAA
GCTCTCATCAACCGTGGCTCCCTCACTTTCTGGCTGGATGATGGGGCGATTCAGGCCTGG
TATGAGTCAGCAACACCTTCTTCACGAGGCAGACCTCAGCGCTAGCGGAGTGTATACTGG
CTTACTATGTTGGCACTGATGAGGGTGTCAGTGAAGTGCTTCATGTGGCAGGAGAAAAA

AGGCTGCACCGGTGCGTCAGCAGAATATGTGATACAGGATATATTCCGCTTCCTCGCTCAC
TGACTCGCTACGCTCGGTCGTTCGACTGCGGCGAGCGGAAATGGCTTACGAACGGGGCG
GAGATTTCCTGGAAGATGCCAGGAAGATACTTAACAGGGAAGTGAGAGGGCCGCGGCA
AAGCCGTTTTTCCATAGGCTCCGCCCCCCTGACAAGCATCACGAAATCTGACGCTCAAAT
CAGTGGTGGCGAAACCCGACAGGACTATAAAGATACCAGGCGTTTCCCCCTGGCGGCTC
CCTCGTGCGCTCTCCTGTTCCTGCCTTTCGGTTTACCGGTGTCATTCCGCTGTTATGGCCG
CGTTTGTCTCATTCCACGCCTGACACTCAGTTCCGGGTAGGCAGTTCGCTCCAAGCTGGA
CTGTATGCACGAACCCCCCGTTCAGTCCGACCGCTGCGCCTTATCCGGTAACTATCGTCTT
GAGTCCAACCCGGAAAGACATGCAAAAGCACCACTGGCAGCAGCCACTGGTAATTGATT
TAGAGGAGTTAGTCTTGAAGTCATGCGCCGGTTAAGGCTAAACTGAAAGGACAAGTTTT
GGTGACTGCGCTCCTCCAAGCCAGTTACCTCGGTTCAAAGAGTTGGTAGCTCAGAGAAC
CTTCGAAAAACCGCCCTGCAAGGCGGTTTTTTCGTTTTCAGAGCAAGAGATTACGCGCA
GACCAAAACGATCTCAAGAAGATCATCTTATTAAGGGGTCTGACGCTCAGTGGAACGAA
AACTCACGTTAAGGGATTTTGGTCATGAGATTATCAAAAAGGATCTTCACCTAGATCCTTT
TAAATTAAAAATGAAGTTTTAAATCAATCTAAAGTATATATGAGTAAACTTGGTCTGACAG
TTACCAATGCTTAATCAGTGAGGCACCTATCTCAGCGATCTGTCTATTTCGTTCATCCATAG
TTGCCTGACTCCCCGTCGTGTAGATAACTACGATACGGGAGGGCTTACCATCTGGCCCCA
GTGCTGCAATGATACCGCGAGACCCACGCTCACCGGCTCCAGATTTATCAGCAATAAACC
AGCCAGCCGGAAGGGCCGAGCGCAGAAGTGGTCCTGCAACTTTATCCGCCTCCATCCAG
TCTATTAATTGTTGCCGGGAAGCTAGAGTAAGTAGTTCGCCAGTTAATAGTTTGCGCAACG
TTGTTGCCATTGCTGCAGGCATCGTGGTGTCACGCTCGTCGTTTGGTATGGCTTCATTCAG
CTCCGGTTCCCAACGATCAAGGCGAGTTACATGATCCCCCATGTTGTGCAAAAAAGCGGT
TAGCTCCTTCGGTCCTCCGATCGTTGTCAGAAGTAAGTTGGCCGCAGTGTTATCACTCATG
GTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTT
GCCCGGCGTCAACACGGGATAATACCGCGCCACATAGCAGAACTTTAAAAGTGCTCATCA
TTGGAAAACGTTCTTCGGGGCGAAAACTCTCAAGGATCTTACCGCTGTTGAGATCCAGTT
CGATGTAACCCACTCGTGCACCCAACTGATCTTCAGCATCTTTTACTTTCACCAGCGTTTC
TGGGTGAGCAAAAACAGGAAGGCAAAATGCCGCAAAAAAGGGAATAAGGGCGACACG
GAAATGTTGAATACTCATACTCTTCCTTTTTCAATATTATTGAAGCATTTATCAGGGTTATT
GTCTCATGAGCGGATACATATTTGAATGTATTTAGAAAAATAAACAAATAGGGGTTCCGCG
CACATTTCCCCGAAAAGTGCCACCTGACGTCTAAGAAACCATTATTATCATGACATTAACC
TATAAAAATAGGCGTATCACGAGGCCCTTTCGTCTTCAAGAATTtctagaggatccctaatacgactcact
atag

The nucleotide sequence of SEQ ID NO.3 is as follows:

ATGggcggcgcatgagagaagcccagaccaattacctacccaaaatggagaaagttcacgttgacatcgaggaagacagcccattcc
tcagagctttgcagcggagcttcccgcagtttgaggtagaagccaagcaggtcactgataatgaccatgctaatgccagagcgttttcgcatctgg
cttcaaaactgatcgaaacggaggtggacccatccgacacgatccttgacattggaagtgcgcccgcccgcagaatgtattctaagcacaagtat
cattgtatctgtccgatgagatgtgcggaagatccggacagattgtataagtatgcaactaagctgaagaaaaactgtaaggaaataactgataag
gaattggacaagaaaatgaaggagctcgccgccgtcatgagcgaccctgacctggaaactgagactatgtgcctccacgacgacgagtcgtgt
cgctacgaagggcaagtcgctgtttaccaggatgtatacgcggttgacggaccgacaagtctctatcaccaagccaataagggagttagagtcg
cctactggataggctttgacaccaccccttttatgtttaagaacttggctggagcatatccatcatactctaccaactgggccgacgaaaccgtgtta
acggctcgtaacataggcctatgcagctctgacgttatggagcggtcacgtagagggatgtccattcttagaaagaagtatttgaaaccatccaac
aatgttctattctctgttggctcgaccatctaccacgagaagagggacttactgaggagctggcacctgccgtctgtatttcacttacgtggcaagca
aaattacacatgtcggtgtgagactatagttagttgcgacgggtacgtcgttaaaagaatagctatcagtccaggcctgtatgggaagccttcaggc
tatgctgctacgatgcaccgcgagggattcttgtgctgcaaagtgacagacacattgaacggggagagggtctcttttcccgtgtgcacgtatgtg
ccagctacattgtgtgaccaaatgactggcatactggcaacagatgtcagtgcggacgacgcgcaaaaactgctggttgggctcaaccagcgtat
agtcgtcaacggtcgcacccagagaaacaccaataccatgaaaaattaccttttgcccgtagtggcccaggcatttgctaggtgggcaaaggaat
ataaggaagatcaagaagatgaaaggccactaggactacgagatagacagttagtcatggggtgttgttgggcttttagaaggcacaagataaca
tctatttataagcgcccggatacccaaaccatcatcaaagtgaacagcgatttccactcattcgtgctgcccaggataggcagtaacacattggaga
tcgggctgagaacaagaatcaggaaaatgttagaggagcacaaggagccgtcacctctcattaccgccgaggacgtacaagaagctaagtgcg
cagccgatgaggctaaggaggtgcgtgaagccgaggagttgcgcgcagctctaccacctttggcagctgatgttgaggagcccactctggaag
ccgatgtcgacttgatgttacaagaggctggggccggctcagtggagacacctcgtggcttgataaaggttaccagctacgctggcgaggacaa
gatcggctcttacgctgtgctttctccgcaggctgtactcaagagtgaaaaattatcttgcatccaccctctcgctgaacaagtcatagtgataacac
actctggccgaaaagggcgttatgccgtggaaccataccatggtaaagtagtggtgccagagggacatgcaatacccgtccaggactttcaagc
tctgagtgaaagtgccaccattgtgtacaacgaacgtgagttcgtaaacaggtacctgcaccatattgccacacatggaggagcgctgaacactg
atgaagaatattacaaaactgtcaagcccagcgagcacgacggcgaatacctgtacgacatcgacaggaaacagtgcgtcaagaaagaactag
tcactgggctagggctcacaggcgagctggtggatcctcccttccatgaattcgcctacgagagtctgagaacacgaccagccgctccttaccaa
gtaccaaccataggggtgtatggcgtgccaggatcaggcaagtctggcatcattaaaagcgcagtcaccaaaaaagatctagtggtgagcgcca
agaaagaaaactgtgcagaaattataagggacgtcaagaaaatgaaagggctggacgtcaatgccagaactgtggactcagtgctcttgaatgg
atgcaaacaccccgtagagaccctgtatattgacgaagcttttgcttgtcatgcaggtactctcagagcgctcatagccattataagacctaaaaag
gcagtgctctgcggggatcccaaacagtgcggtttttttaacatgatgtgcctgaaagtgcattttaaccacgagatttgcacacaagtcttccacaa
aagcatctctcgccgttgcactaaatctgtgacttcggtcgtctcaaccttgttttacgacaaaaaaatgagaacgacgaatccgaaagagactaag
attgtgattgacactaccggcagtaccaaacctaagcaggacgatctcattctcacttgtttcagagggtgggtgaagcagttgcaaatagattaca
aaggcaacgaaataatgacggcagctgcctctcaagggctgacccgtaaaggtgtgtatgccgttcggtacaaggtgaatgaaaatcctctgtac
gcacccacctcagaacatgtgaacgtcctactgacccgcacggaggaccgcatcgtgtggaaaacactagccggcgacccatggataaaaac
actgactgccaagtaccctgggaatttcactgccacgatagaggagtggcaagcagagcatgatgccatcatgaggcacatcttggagagaccg
gaccctaccgacgtcttccagaataaggcaaacgtgtgttgggccaaggctttagtgccggtgctgaagaccgctggcatagacatgaccactg

aacaatggaacactgtggattattttgaaacggacaaagctcactcagcagagatagtattgaaccaactatgcgtgaggttctttggactcgatctg
gactccggtctattttctgcacccactgttccgttatccattaggaataatcactgggataactccccgtcgcctaacatgtacgggctgaataaaga
agtggtccgtcagctctctcgcaggtacccacaactgcctcgggcagttgccactggaagagtctatgacatgaacactggtacactgcgcaatta
tgatccgcgcataaacctagtacctgtaaacagaagactgcctcatgctttagtcctccaccataatgaacacccacagagtgacttttcttcattcgt
cagcaaattgaagggcagaactgtcctggtggtcggggaaaagttgtccgtcccaggcaaaatggttgactggttgtcagaccggcctgaggct
accttcagagctcggctggatttaggcatcccaggtgatgtgcccaaatatgacataatatttgttaatgtgaggaccccatataaataccatcactat
cagcagtgtgaagaccatgccattaagcttagcatgttgaccaagaaagcttgtctgcatctgaatcccggcggaacctgtgtcagcataggttatg
gttacgctgacagggccagcgaaagcatcattggtgctatagcgcggcagttcaagttttcccgggtatgcaaaccgaaatcctcacttgaagag
acggaagttctgtttgtattcattgggtacgatcgcaaggcccgtacgcacaatccttacaagctttcatcaaccttgaccaacatttatacaggttcc
agactccacgaagccggatgtgcaccctcatatcatgtggtgcgaggggatattgccacggccaccgaaggagtgattataaatgctgctaaca
gcaaaggacaacctggcggaggggtgtgcggagcgctgtataagaagttcccggaaagcttcgatttacagccgatcgaagtaggaaaagcg
cgactggtcaaaggtgcagctaaacatatcattcatgccgtaggaccaaacttcaacaaagtttcggaggttgaaggtgacaaacagttggcaga
ggcttatgagtccatcgctaagattgtcaacgataacaattacaagtcagtagcgattccactgttgtccaccggcatcttttccgggaacaaagatc
gactaacccaatcattgaaccatttgctgacagctttagacaccactgatgcagatgtagccatatactgcagggacaagaaatgggaaatgactc
tcaaggaagcagtggctaggagagaagcagtggaggagatatgcatatccgacgactcttcagtgacagaacctgatgcagagctggtgaggg
tgcatccgaagagttctttggctggaaggaagggctacagcacaagcgatggcaaaactttctcatatttggaagggaccaagtttcaccaggcg
gccaaggatatagcagaaattaatgccatgtggcccgttgcaacggaggccaatgagcaggtatgcatgtatatcctcggagaaagcatgagca
gtattaggtcgaaatgccccgtcgaagagtcggaagcctcctcaccacctagcacgctgccttgcttgtgcatccatgccatgactccagaaaga
gtacagcgcctaaaagcctcacgtccagaacaaattactgtgtgctcatcctttccattgccgaagtatagaatcactggtgtgcagaagatccaat
gctcccagcctatattgttctcaccgaaagtgcctgcgtatattcatccaaggaagtatctcgtggaaacaccaccggtagacgagactccggagc
catcggcagagaaccaatccacagaggggacacctgaacaaccaccacttataaccgaggatgagaccaggactagaacgcctgagccgatc
atcatcgaagaggaagaagaggatagcataagtttgctgtcagatggcccgacccaccaggtgctgcaagtcgaggcagacattcacgggccg
ccctctgtatctagctcatcctggtccattcctcatgcatccgactttgatgtggacagtttatccatacttgacaccctggagggagctagcgtgacc
agcggggcaacgtcagccgagactaactcttacttcgcaaagagtatggagtttctggcgcgaccggtgcctgcgcctcgaacagtattcagga
accctccacatcccgctccgcgcacaagaacaccgtcacttgcacccagcagggcctgctcgagaaccagcctagtttccaccccgccaggcg
tgaatagggtgatcactagagaggagctcgaggcgcttaccccgtcacgcactcctagcaggtcggtctcgagaaccagcctggtctccaaccc
gccaggcgtaaatagggtgattacaagagaggagtttgaggcgttcgtagcacaacaacaatgacggtttgatgcgggtgcatacatcttttcctc
cgacaccggtcaagggcatttacaacaaaaatcagtaaggcaaacggtgctatccgaagtggtgttggagaggaccgaattggagatttcgtatg
ccccgcgcctcgaccaagaaaaagaagaattactacgcaagaaattacagttaaatcccacacctgctaacagaagcagataccagtccaggaa
ggtggagaacatgaaagccataacagctagacgtattctgcaaggcctagggcattatttgaaggcagaaggaaaagtggagtgctaccgaacc
ctgcatcctgttcctttgtattcatctagtgtgaaccgtgccttctcaagccccaaggtcgcagtggaagcctgtaacgccatgttgaaagagaacttt
ccgactgtggcttcttactgtattattccagagtacgatgcctatttggacatggttgacggagcttcatgctgcttagacactgccagtttttgccctg
caaagctgcgcagctttccaaagaaacactcctatttggaacccacaatacgatcggcagtgccttcagcgatccagaacacgctccagaacgtc
ctggcagctgccacaaaaagaaattgcaatgtcacgcaaatgagagaattgcccgtattggattcggcggcctttaatgtggaatgcttcaagaaa

tatgcgtgtaataatgaatattgggaaacgtttaaagaaaaccccatcaggcttactgaagaaaacgtggtaaattacattaccaaattaaaaggac
caaaagctgctgctctttttgcgaagacacataatttgaatatgttgcaggacataccaatggacaggtttgtaatggacttaaagagagacgtgaaa
gtgactccaggaacaaaacatactgaagaacggcccaaggtacaggtgatccaggctgccgatccgctagcaacagcgtatctgtgcggaatc
caccgagagctggttaggagattaaatgcggtcctgcttccgaacattcatacactgtttgatatgtcggctgaagactttgacgctattatagccga
gcacttccagcctggggattgtgttctggaaactgacatcgcgtcgtttgataaaagtgaggacgacgccatggctctgaccgcgttaatgattctg
gaagacttaggtgtggacgcagagctgttgacgctgattgaggcggctttcggcgaaatttcatcaatacatttgcccactaaaactaaatttaaatt
cggagccatgatgaaatctggaatgttcctcacactgtttgtgaacacagtcattaacattgtaatcgcaagcagagtgttgagagaacggctaacc
ggatcaccatgtgcagcattcattggagatgacaatatcgtgaaaggagtcaaatcggacaaattaatggcagacaggtgcgccacctggttgaa
tatggaagtcaagattatagatgctgtggtgggcgagaaagcgccctatttctgtggagggtttattttgtgtgactccgtgaccggcacagcgtgc
cgtgtggcagaccccctaaaaaggctgtttaagcttggcaaacctctggcagcagacgatgaacatgatgatgacaggagaagggcattgcatg
aagagtcaacacgctggaaccgagtgggtattctttcagagctgtgcaaggcagtagaatcaaggtatgaaaccgtaggaacttccatcatagtta
tggccatgactactctagctagcagtgttaaatcattcagctacctgagaggggcccctataactctctacggctaacctgaatggactacgacatc
gatggcgcgccACC

The nucleotide sequence of SEQ ID NO.4 is as follows:

ATGGTGAGCAAGGGCGAGGAGCTGTTCACCGGGGTGGTGCCCATCCTGGTCGAGCT
GGACGGCGACGTAAACGGCCACAAGTTCAGCGTGTCCGGCGAGGGCGAGGGCGATGCC
ACCTACGGCAAGCTGACCCTGAAGTTCATCTGCACCACCGGCAAGCTGCCCGTGCCCTG
GCCCACCCTCGTGACCACCCTGACCTACGGCGTGCAGTGCTTCAGCCGCTACCCCGACC
ACATGAAGCAGCACGACTTCTTCAAGTCCGCCATGCCCGAAGGCTACGTCCAGGAGCGC
ACCATCTTCTTCAAGGACGACGGCAACTACAAGACCCGCGCCGAGGTGAAGTTCGAGGG
CGACACCCTGGTGAACCGCATCGAGCTGAAGGGCATCGACTTCAAGGAGGACGGCAAC
ATCCTGGGGCACAAGCTGGAGTACAACTACAACAGCCACAACGTCTATATCATGGCCGAC
AAGCAGAAGAACGGCATCAAGGTGAACTTCAAGATCCGCCACAACATCGAGGACGGCA
GCGTGCAGCTCGCCGACCACTACCAGCAGAACACCCCCATCGGCGACGGCCCCGTGCTG
CTGCCCGACAACCACTACCTGAGCACCCAGTCCGCCCTGAGCAAAGACCCCAACGAGA
AGCGCGATCACATGGTCCTGCTGGAGTTCGTGACCGCCGCCGGGATCACTCTCGGCATG
GACGAGCTGTACAAG

The nucleotide sequence of SEQ ID NO.5 is as follows:

GTCGACATTGATTATTGACTAGTTATTAATAGTAATCAATTACGGGGTCATTAGTTCAT
AGCCCATATATGGAGTTCCGCGTTACATAACTTACGGTAAATGGCCCGCCTGGCTGACCGC
CCAACGACCCCCGCCCATTGACGTCAATAATGACGTATGTTCCCATAGTAACGCCAATAG
GGACTTTCCATTGACGTCAATGGGTGGACTATTTACGGTAAACTGCCCACTTGGCAGTAC
ATCAAGTGTATCATATGCCAAGTACGCCCCCTATTGACGTCAATGACGGTAAATGGCCCGC
CTGGCATTATGCCCAGTACATGACCTTATGGGACTTTCCTACTTGGCAGTACATCTACGTAT

TAGTCATCGCTATTACCATGGGTCGAGGTGAGCCCCACGTTCTGCTTCACTCTCCCCATCT
CCCCCCCCTCCCCACCCCCAATTTTGTATTTATTTATTTTTTAATTATTTTGTGCAGCGATGG
GGGCGGGGGGGGGGGGGGCGCGCGCCAGGCGGGGCGGGGCGGGGCGAGGGGCGGGG
CGGGGCGAGGCGGAGAGGTGCGGCGGCAGCCAATCAGAGCGGCGCGCTCCGAAAGTTT
CCTTTTATGGCGAGGCGGCGGCGGCGGCGGCCCTATAAAAAGCGAAGCGCGCGGCGGGC
GGGAGTCGCTGCGTTGCCTTCGCCCCGTGCCCCGCTCCGCGCCGCCTCGCGCCGCCCGC
CCCGGCTCTGACTGACCGCGTTACTCCCACAGGTGAGCGGGCGGGACGGCCCTTCTCCT
CCGGGCTGTAATTAGCGCTTGGTTTAATGACGGCTCGTTTCTTTTCTGTGGCTGCGTGAAA
GCCTTAAAGGGCTCCGGGAGGGCCCTTTGTGCGGGGGGGAGCGGCTCGGGGGGTGCGT
GCGTGTGTGTGTGCGTGGGGAGCGCCGCGTGCGGCCCGCGCTGCCCGGCGGCTGTGAG
CGCTGCGGGCGCGGCGCGGGGCTTTGTGCGCTCCGCGTGTGCGCGAGGGGAGCGCGGC
CGGGGGCGGTGCCCCGCGGTGCGGGGGGGCTGCGAGGGGAACAAAGGCTGCGTGCGG
GGTGTGTGCGTGGGGGGGTGAGCAGGGGGTGTGGGCGCGGCGGTCGGGCTGTAACCCC
CCCCTGCACCCCCCTCCCCGAGTTGCTGAGCACGGCCCGGCTTCGGGTGCGGGGCTCCG
TGCGGGGCGTGGCGCGGGGCTCGCCGTGCCGGGCGGGGGGTGGCGGCAGGTGGGGGTG
CCGGGCGGGGCGGGGCCGCCTCGGGCCGGGGAGGGCTCGGGGGAGGGGCGCGGCGGC
CCCGGAGCGCCGGCGGCTGTCGAGGCGCGGCGAGCCGCAGCCATTGCCTTTTATGGTAA
TCGTGCGAGAGGGCGCAGGGACTTCCTTTGTCCCAAATCTGTGCGGAGCCGAAATCTGG
GAGGCGCCGCCGCACCCCCTCTAGCGGGCGCGGGGCGAAGCGGTGCGGCGCCGGCAGG
AAGGAAATGGGCGGGGAGGGCCTTCGTGCGTCGCCGCGCCGCCGTCCCCTTCTCCCTCT
CCAGCCTCGGGGCTGTCCGCGGGGGGACGGCTGCCTTCGGGGGGGACGGGGCAGGGCG
GGGTTCGGCTTCTGGCGTGTGACCGGCGGCTCTAGAGCCTCTGCTAACCATGTTCATGCC
TTCTTCTTTTTCCTACAGCTCCTGGGCAACGTGCTGGTTATTGTGCTGTCTCATCATTTTGG
CAAAGAATTCGGCGGCCGCATGAGTCTGGCCATTCCAGTTATGTGCCTGCTGGCAAATAC
CACGTTCCCCTGCTCCCAGCCCCCTTGCACACCCTGCTGCTACGAAAAAGAGCCGGAGA
AAACCTTGCGCATGCTTGAAGACAATGTCATGAGCCCCGGGTACTATCAGCTGCTACAAG
CATCCTTAACATGTTCTCCCCGACGCCAGCGGCGCAGTATTAAGGACCACTTCAATGTCTA
TAAAGCCACAAGACCGTACCTAGCTCACTGTCCCGACTGTGGAGAAGGGCACTCGTGCC
ATAGTCCCGTAGCGCTAGAACGCATCAGAAACGAAGCGACAGACGGGACGTTGAAAATC
CAGGTTTCCTTGCAAATCGGAATAAAGACGGATGATAGCCATGATTGGACCAAGCTGCGT
TATATGGACAATCACATGCCAGCAGACGCAGAGCGGGCCGGGCTATTTGTAAGAACGTCA
GCACCGTGCACGATTACTGGAACAATGGGACACTTCATTCTGGCCCGATGTCCGAAAGG
AGAAACTCTGACGGTAGGGTTCACTGACGGTAGGAAGATCAGTCACTCATGTACGCACC
CATTTCACCATGACCCTCCTGTGATAGGCCGGGAAAAATTCCATTCCCGACCGCAGCACG

GTAGGGAACTACCTTGCAGCACGTACGCGCAGAGCACCGCTGCAACTGCCGAGGAGATA
GAGGTACATATGCCCCCAGACACCCCAGATCGCACATTAATGTCACAACAGTCCGGCAAT
GTAAAGATCACAGTCAATAGTCAGACGGTGCGGTACAAGTGCAATTGTGGTGACTCAAG
TGAAGGATTAACCACTACAGATAAAGTGATTAATAACTGCAAGGTTGATCAATGCCATGC
CGCGGTCACCAATCACAAAAAATGGCAGTATAATTCCCCTCTGGTCCCGCGTAATGCTGA
ATTCGGGGACCGGAAAGGAAAAGTTCACATTCCATTTCCTCTGGCAAATGTGACATGCA
GGGTGCCTAAAGCAAGAAACCCCACCGTGACGTACGGAAAAAACCAAGTCATCATGTTG
CTGTATCCTGACCACCCAACGCTCCTGTCCTACAGGAATATGGGAGAAGAACCAAACTAT
CAAGAAGAGTGGGTGACGCATAAGAAGGAGATCAGGTTAACCGTGCCGACTGAAGGGC
TCGAGGTCACGTGGGGTAACAATGAGCCGTACAAGTATTGGCCGCAGTTATCCACAAAC
GGTACAGCCCACGGCCACCCGCATGAGATAATTCTGTATTATTATGAGCTGTACCCAACTA
TGACTGTGGTAGTTTTGTCAGTGGCCTCGTTCATACTCCTGTCGATGGTGGGTGTGGCAG
TGGGGATGTGCATGTGTGCACGACGCAGATGCATTACACCGTACGAACTGACACCAGGA
GCTACCGTCCCTTTCCTGCTTAGCCTAATATGCTGCATTAGAACAGCTAAAGCGGCCACAT
ACCAGGAGGCCGCGGTATACCTGTGGAACGAGCAGCAGCCTTTATTTTGGCTGCAAGCC
CTTATTCCGCTGGCAGCCCTGATTGTCCTATGTAACTGTCTGAGACTCTTACCATGCTGTT
GTAAAATGTTGACTTTTTTAGCCGTACTGAGCGTCGGTGCCCACACTGTGAGTGCGTACG
AACACGTAACAGTGATCCCGAACACGGTGGGAGTACCGTATAAGACTCTAGTCAACAGA
CCGGGCTACAGCCCCATGGTATTGGAGATGGAGCTTCTGTCTGTCACCTTGGAACCAACG
CTATCGCTTGATTACATCACGTGCGAGTATAAAACCGTTATCCCGTCTCCGTACGTGAAAT
GCTGCGGTACAGCAGAGTGTAAGGACAAGAGCCTACCTGATTACAGCTGTAAGGTCTTC
ACCGGCGTCTACCCATTCATGTGGGGCGGCGCCTACTGCTTCTGCGACACCGAAAATACG
CAATTGAGCGAAGCACATGTGGAGAAGTCCGAATCATGCAAAACAGAATTTGCATCAGC
ATACAGGGCTCATACCGCATCCGCGTCAGCTAAGCTCCGCGTCCTTTACCAAGGAAATAA
TATCACTGTAGCTGCTTATGCAAACGGCGACCATGCCGTCACAGTTAAGGACGCTAAATT
CATAGTGGGGCCAATGTCTTCAGCCTGGACACCTTTCGACAATAAAATCGTGGTGTACAA
AGGCGACGTCTACAACATGGACTACCCGCCCTTCGGCGCAGGAAGACCAGGACAATTTG
GCGACATCCAAAGTCGCACGCCTGAGAGCGAAGACGTCTATGCTAATACACAACTGGTA
CTGCAGAGACCGTCCGCGGGTACGGTGCACGTGCCGTACTCTCAGGCACCATCTGGCTT
CAAGTATTGGCTAAAAGAACGAGGGGCGTCGCTGCAGCACACAGCACCATTTGGCTGTC
AAATAGCAACAAACCCGGTAAGAGCGATGAACTGCGCCGTAGGGAACATGCCTATCTCC
ATCGACATACCGGACGCGGCCTTTACCAGGGTCGTCGACGCGCCATCTTTAACGGACATG
TCGTGTGAGGTATCAGCCTGCACCCATTCCTCAGACTTTGGGGGCGTAGCCATCATTAAAT
ATGCAGCCAGTAAGAAAGGCAAGTGTGCAGTGCACTCGATGACTAACGCCGTCACTATT

CGGGAAGCTGAAATAGAAGTAGAAGGGAACTCTCAGTTGCAAATCTCTTTTTCGACGGC
CCTAGCCAGCGCCGAATTTCGCGTACAAGTCTGTTCTACACAAGTACACTGTGCAGCCGA
GTGCCATCCACCGAAAGACCATATAGTCAATTACCCGGCGTCACACACCACCCTCGGGGT
CCAAGACATTTCCGCTACGGCGATGTCATGGGTGCAGAAGATCACGGGAGGTGTGGGAC
TGGTTGTCGCTGTTGCAGCACTGATCCTAATCGTGGTGCTATGCGTGTCGTTTAGCAGGC
ACTAAGCTAGCAGATCTTTTTCCCTCTGCCAAAAATTATGGGGACATCATGAAGCCCCTTG
AGCATCTGACTTCTGGCTAATAAAGGAAATTTATTTTCATTGCAATAGTGTGTTGGAATTT
TTTGTGTCTCTCACTCGGAAGGACATATGGGAGGGCAAATCATTTAAAACATCAGAATGA
GTATTTGGTTTAGAGTTTGGCAACATATGCCCATATGCTGGCTGCCATGAACAAAGGTTGG
CTATAAAGAGGTCATCAGTATATGAAACAGCCCCCTGCTGTCCATTCCTTATTCCATAGAA
AAGCCTTGACTTGAGGTTAGATTTTTTTTATATTTTGTTTTGTGTTATTTTTTTCTTTAACAT
CCCTAAAATTTTCCTTACATGTTTTACTAGCCAGATTTTTCCTCCTCTCCTGACTACTCCCA
GTCATAGCTGTCCCTCTTCTCTTATGGAGATCCCTCGACCTGCAGCCCAAGCTTGGCGTAA
TCATGGTCATAGCTGTTTCCTGTGTGAAATTGTTATCCGCTCACAATTCCACACAACATAC
GAGCCGGAAGCATAAAGTGTAAAGCCTGGGGTGCCTAATGAGTGAGCTAACTCACATTA
ATTGCGTTGCGCTCACTGCCCGCTTTCCAGTCGGGAAACCTGTCGTGCCAGCGGATCCGC
ATCTCAATTAGTCAGCAACCATAGTCCCGCCCCTAACTCCGCCCATCCCGCCCCTAACTCC
GCCCAGTTCCGCCCATTCTCCGCCCCATGGCTGACTAATTTTTTTTATTTATGCAGAGGCC
GAGGCCGCCTCGGCCTCTGAGCTATTCCAGAAGTAGTGAGGAGGCTTTTTTGGAGGCCT
AGGCTTTTGCAAAAAGCTAACTTGTTTATTGCAGCTTATAATGGTTACAAATAAAGCAATA
GCATCACAAATTTCACAAATAAAGCATTTTTTTCACTGCATTCTAGTTGTGGTTTGTCCAA
ACTCATCAATGTATCTTATCATGTCTGGATCCGCTGCATTAATGAATCGGCCAACGCGCGG
GGAGAGGCGGTTTGCGTATTGGGCGCTCTTCCGCTTCCTCGCTCACTGACTCGCTGCGCT
CGGTCGTTCGGCTGCGGCGAGCGGTATCAGCTCACTCAAAGGCGGTAATACGGTTATCCA
CAGAATCAGGGGATAACGCAGGAAAGAACATGTGAGCAAAAGGCCAGCAAAAGGCCAG
GAACCGTAAAAAGGCCGCGTTGCTGGCGTTTTTCCATAGGCTCCGCCCCCCTGACGAGC
ATCACAAAAATCGACGCTCAAGTCAGAGGTGGCGAAACCCGACAGGACTATAAAGATAC
CAGGCGTTTCCCCCTGGAAGCTCCCTCGTGCGCTCTCCTGTTCCGACCCTGCCGCTTACC
GGATACCTGTCCGCCTTTCTCCCTTCGGGAAGCGTGGCGCTTTCTCAATGCTCACGCTGT
AGGTATCTCAGTTCGGTGTAGGTCGTTCGCTCCAAGCTGGGCTGTGTGCACGAACCCCCC
GTTCAGCCCGACCGCTGCGCCTTATCCGGTAACTATCGTCTTGAGTCCAACCCGGTAAGA
CACGACTTATCGCCACTGGCAGCAGCCACTGGTAACAGGATTAGCAGAGCGAGGTATGT
AGGCGGTGCTACAGAGTTCTTGAAGTGGTGGCCTAACTACGGCTACACTAGAAGGACAG
TATTTGGTATCTGCGCTCTGCTGAAGCCAGTTACCTTCGGAAAAAGAGTTGGTAGCTCTT

GATCCGGCAAACAAACCACCGCTGGTAGCGGTGGTTTTTTTGTTTGCAAGCAGCAGATTA
CGCGCAGAAAAAAAGGATCTCAAGAAGATCCTTTGATCTTTTCTACGGGGTCTGACGCTC
AGTGGAACGAAAACTCACGTTAAGGGATTTTGGTCATGAGATTATCAAAAAGGATCTTCA
CCTAGATCCTTTTAAATTAAAAATGAAGTTTTAAATCAATCTAAAGTATATATGAGTAAACT
TGGTCTGACAGTTACCAATGCTTAATCAGTGAGGCACCTATCTCAGCGATCTGTCTATTTC
GTTCATCCATAGTTGCCTGACTCCCCGTCGTGTAGATAACTACGATACGGGAGGGCTTACC
ATCTGGCCCCAGTGCTGCAATGATACCGCGAGACCCACGCTCACCGGCTCCAGATTTATC
AGCAATAAACCAGCCAGCCGGAAGGGCCGAGCGCAGAAGTGGTCCTGCAACTTTATCCG
CCTCCATCCAGTCTATTAATTGTTGCCGGGAAGCTAGAGTAAGTAGTTCGCCAGTTAATAG
TTTGCGCAACGTTGTTGCCATTGCTACAGGCATCGTGGTGTCACGCTCGTCGTTTGGTATG
GCTTCATTCAGCTCCGGTTCCCAACGATCAAGGCGAGTTACATGATCCCCCATGTTGTGC
AAAAAAGCGGTTAGCTCCTTCGGTCCTCCGATCGTTGTCAGAAGTAAGTTGGCCGCAGT
GTTATCACTCATGGTTATGGCAGCACTGCATAATTCTCTTACTGTCATGCCATCCGTAAGAT
GCTTTTCTGTGACTGGTGAGTACTCAACCAAGTCATTCTGAGAATAGTGTATGCGGCGAC
CGAGTTGCTCTTGCCCGGCGTCAATACGGGATAATACCGCGCCACATAGCAGAACTTTAA
AAGTGCTCATCATTGGAAAACGTTCTTCGGGGCGAAAACTCTCAAGGATCTTACCGCTGT
TGAGATCCAGTTCGATGTAACCCACTCGTGCACCCAACTGATCTTCAGCATCTTTTACTTT
CACCAGCGTTTCTGGGTGAGCAAAAACAGGAAGGCAAAATGCCGCAAAAAAGGGAATA
AGGGCGACACGGAAATGTTGAATACTCATACTCTTCCTTTTTCAATATTATTGAAGCATTTA
TCAGGGTTATTGTCTCATGAGCGGATACATATTTGAATGTATTTAGAAAAATAAACAAATA
GGGGTTCCGCGCACATTTCCCCGAAAAGTGCCACCTG

Subsequently, a series of expression plasmids of different viral envelope proteins were constructed (pcaggs-CHIKV-E, VEEV-E, EBOV-GP, NIV-GF, LASV-GP, RABV-G, H7N1-HANA, and RSV-F). The sequence of Pcaggs-CHIKV-E is shown in SEQ ID NO.5, wherein the sequence of the pcaggs vector is shown in SEQ ID NO.6, and the sequence of CHIKV-E is shown in SEQ ID NO.7. The construction method is as follows: in the multiple cloning site of the pcaggs vector (preserved in our laboratory), the synthesized CHIKV-E fragment was inserted using NotI and NheI restriction enzyme sites to obtain the Pcaggs-CHIKV-E clone. Other expression plasmids of viral envelope proteins were constructed in the same way. The vector sequence is shown in SEQ ID NO.6, the VEEV-E sequence is shown in SEQ ID NO.8, the EBOV-GP sequence is shown in SEQ ID NO.9, the NIV-GF sequence is shown in SEQ ID NO.10, the LASV-GP sequence is shown in SEQ ID NO.11, the RABV-G sequence is shown in SEQ ID NO.12, the H7N1-HANA sequence is shown in SEQ ID NO.13, and the RSV-F sequence is shown in SEQ ID NO.14.

Subsequently, the packaging efficiency and the expression of target proteins of different viral envelope proteins were verified. The specific experimental process is as follows: firstly, 2.5×10⁵ BHK-21 cells (preserved in our laboratory) were inoculated in a 6-well plate and cultured overnight at 37°C. The expression plasmids of corresponding viral envelope proteins were transfected into BHK-21 cells using fugene liposomes. The transfection system consisted of 4 µl fugene liposomes mixed with 1 µg envelope protein expression plasmids. After standing for 15 minutes, the mixture was dropped into the BHK-21 cells cultured overnight. After 12 hours, VEEVrep-EGFP saRNA was transfected using Dmrie-C liposomes. The transfection system was prepared by adding 4 µl Dmrie-C liposomes and 1 µg RNA to 1 ml OPTI-MEM medium. After mixing, the original medium in BHK-21 cells was replaced with this mixture. After 4-6 hours, it was replaced with fresh 2% FBS DMEM medium. The expression of EGFP was observed at different time points after RNA transfection, and the cell culture supernatant was collected at the 48-hour time point, aliquoted and stored at -80°C. Subsequently, the collected 48-hour cell culture supernatant was used to verify the delivery effect on naive BHK-21 cells, and the expression of EGFP was observed 24 hours after delivery. The specific experimental process is as follows: firstly, 2.5×10⁵ BHK-21 cells were inoculated in a 6-well plate. After overnight culture at 37°C, the original medium was replaced with 2% FBS DMEM medium, and 500 µl of the aforementioned collected 48-hour post-transfection cell culture supernatant was added. The expression of EGFP was observed using a fluorescence microscope after 24 hours.

### Experimental Results

It was found that VEEVrep-EGFP saRNA could effectively express the target protein EGFP after transfecting cells, which proved the ability of the alphavirus genome to self-replicate and express exogenous genes. Further observation showed that the supernatants collected after co-transfection of saRNA with the expression plasmids constructed by the envelope proteins of these 8 different viruses could effectively deliver saRNA and express the target protein EGFP after infecting naive BHK-21 cells (as shown in FIG. 2). Based on the above results, it is inferred that the co-transfection of VEEVrep-EGFP saRNA with the expression plasmids constructed by the envelope proteins of different viruses can form microvesicles, which can effectively deliver saRNA and express the target protein EGFP. That is, the VRV delivery platform constructed in the present disclosure can achieve effective delivery of saRNA and effective expression of target proteins.

The nucleotide sequence of SEQ ID NO.6 is as follows:

GTCGACATTGATTATTGACTAGTTATTAATAGTAATCAATTACGGGGTCATTAGTTCAT
AGCCCATATATGGAGTTCCGCGTTACATAACTTACGGTAAATGGCCCGCCTGGCTGACCGC
CCAACGACCCCCGCCCATTGACGTCAATAATGACGTATGTTCCCATAGTAACGCCAATAG
GGACTTTCCATTGACGTCAATGGGTGGACTATTTACGGTAAACTGCCCACTTGGCAGTAC

ATCAAGTGTATCATATGCCAAGTACGCCCCCTATTGACGTCAATGACGGTAAATGGCCCGC
CTGGCATTATGCCCAGTACATGACCTTATGGGACTTTCCTACTTGGCAGTACATCTACGTAT
TAGTCATCGCTATTACCATGGGTCGAGGTGAGCCCCACGTTCTGCTTCACTCTCCCCATCT
CCCCCCCCTCCCCACCCCCAATTTTGTATTTATTTATTTTTTAATTATTTTGTGCAGCGATGG
GGGCGGGGGGGGGGGGGGCGCGCGCCAGGCGGGGCGGGGCGGGGCGAGGGGCGGGG
CGGGGCGAGGCGGAGAGGTGCGGCGGCAGCCAATCAGAGCGGCGCGCTCCGAAAGTTT
CCTTTTATGGCGAGGCGGCGGCGGCGGCGGCCCTATAAAAAGCGAAGCGCGCGGCGGGC
GGGAGTCGCTGCGTTGCCTTCGCCCCGTGCCCCGCTCCGCGCCGCCTCGCGCCGCCCGC
CCCGGCTCTGACTGACCGCGTTACTCCCACAGGTGAGCGGGCGGGACGGCCCTTCTCCT
CCGGGCTGTAATTAGCGCTTGGTTTAATGACGGCTCGTTTCTTTTCTGTGGCTGCGTGAAA
GCCTTAAAGGGCTCCGGGAGGGCCCTTTGTGCGGGGGGGAGCGGCTCGGGGGGTGCGT
GCGTGTGTGTGTGCGTGGGGAGCGCCGCGTGCGGCCCGCGCTGCCCGGCGGCTGTGAG
CGCTGCGGGCGCGGCGCGGGGCTTTGTGCGCTCCGCGTGTGCGCGAGGGGAGCGCGGC
CGGGGGCGGTGCCCCGCGGTGCGGGGGGGCTGCGAGGGGAACAAAGGCTGCGTGCGG
GGTGTGTGCGTGGGGGGGTGAGCAGGGGGTGTGGGCGCGGCGGTCGGGCTGTAACCCC
CCCCTGCACCCCCCTCCCCGAGTTGCTGAGCACGGCCCGGCTTCGGGTGCGGGGCTCCG
TGCGGGGCGTGGCGCGGGGCTCGCCGTGCCGGGCGGGGGGTGGCGGCAGGTGGGGGTG
CCGGGCGGGGCGGGGCCGCCTCGGGCCGGGGAGGGCTCGGGGGAGGGGCGCGGCGGC
CCCGGAGCGCCGGCGGCTGTCGAGGCGCGGCGAGCCGCAGCCATTGCCTTTTATGGTAA
TCGTGCGAGAGGGCGCAGGGACTTCCTTTGTCCCAAATCTGTGCGGAGCCGAAATCTGG
GAGGCGCCGCCGCACCCCCTCTAGCGGGCGCGGGGCGAAGCGGTGCGGCGCCGGCAGG
AAGGAAATGGGCGGGGAGGGCCTTCGTGCGTCGCCGCGCCGCCGTCCCCTTCTCCCTCT
CCAGCCTCGGGGCTGTCCGCGGGGGGACGGCTGCCTTCGGGGGGGACGGGGCAGGGCG
GGGTTCGGCTTCTGGCGTGTGACCGGCGGCTCTAGAGCCTCTGCTAACCATGTTCATGCC
TTCTTCTTTTTCCTACAGCTCCTGGGCAACGTGCTGGTTATTGTGCTGTCTCATCATTTTGG
CAAAGAATTCGGCGGCCGCGCTAGCAGATCTTTTTCCCTCTGCCAAAAATTATGGGGACA
TCATGAAGCCCCTTGAGCATCTGACTTCTGGCTAATAAAGGAAATTTATTTTCATTGCAAT
AGTGTGTTGGAATTTTTTGTGTCTCTCACTCGGAAGGACATATGGGAGGGCAAATCATTT
AAAACATCAGAATGAGTATTTGGTTTAGAGTTTGGCAACATATGCCCATATGCTGGCTGCC
ATGAACAAAGGTTGGCTATAAAGAGGTCATCAGTATATGAAACAGCCCCCTGCTGTCCAT
TCCTTATTCCATAGAAAAGCCTTGACTTGAGGTTAGATTTTTTTTATATTTTGTTTTGTGTTA
TTTTTTTCTTTAACATCCCTAAAATTTTCCTTACATGTTTTACTAGCCAGATTTTTCCTCCTC
TCCTGACTACTCCCAGTCATAGCTGTCCCTCTTCTCTTATGGAGATCCCTCGACCTGCAGC
CCAAGCTTGGCGTAATCATGGTCATAGCTGTTTCCTGTGTGAAATTGTTATCCGCTCACAA

TTCCACACAACATACGAGCCGGAAGCATAAAGTGTAAAGCCTGGGGTGCCTAATGAGTG
AGCTAACTCACATTAATTGCGTTGCGCTCACTGCCCGCTTTCCAGTCGGGAAACCTGTCG
TGCCAGCGGATCCGCATCTCAATTAGTCAGCAACCATAGTCCCGCCCCTAACTCCGCCCA
TCCCGCCCCTAACTCCGCCCAGTTCCGCCCATTCTCCGCCCCATGGCTGACTAATTTTTTT
TATTTATGCAGAGGCCGAGGCCGCCTCGGCCTCTGAGCTATTCCAGAAGTAGTGAGGAGG
CTTTTTTGGAGGCCTAGGCTTTTGCAAAAAGCTAACTTGTTTATTGCAGCTTATAATGGTT
ACAAATAAAGCAATAGCATCACAAATTTCACAAATAAAGCATTTTTTTCACTGCATTCTAG
TTGTGGTTTGTCCAAACTCATCAATGTATCTTATCATGTCTGGATCCGCTGCATTAATGAAT
CGGCCAACGCGCGGGGAGAGGCGGTTTGCGTATTGGGCGCTCTTCCGCTTCCTCGCTCA
CTGACTCGCTGCGCTCGGTCGTTCGGCTGCGGCGAGCGGTATCAGCTCACTCAAAGGCG
GTAATACGGTTATCCACAGAATCAGGGGATAACGCAGGAAAGAACATGTGAGCAAAAGG
CCAGCAAAAGGCCAGGAACCGTAAAAAGGCCGCGTTGCTGGCGTTTTTCCATAGGCTCC
GCCCCCCTGACGAGCATCACAAAAATCGACGCTCAAGTCAGAGGTGGCGAAACCCGAC
AGGACTATAAAGATACCAGGCGTTTCCCCCTGGAAGCTCCCTCGTGCGCTCTCCTGTTCC
GACCCTGCCGCTTACCGGATACCTGTCCGCCTTTCTCCCTTCGGGAAGCGTGGCGCTTTC
TCAATGCTCACGCTGTAGGTATCTCAGTTCGGTGTAGGTCGTTCGCTCCAAGCTGGGCTG
TGTGCACGAACCCCCCGTTCAGCCCGACCGCTGCGCCTTATCCGGTAACTATCGTCTTGA
GTCCAACCCGGTAAGACACGACTTATCGCCACTGGCAGCAGCCACTGGTAACAGGATTA
GCAGAGCGAGGTATGTAGGCGGTGCTACAGAGTTCTTGAAGTGGTGGCCTAACTACGGC
TACACTAGAAGGACAGTATTTGGTATCTGCGCTCTGCTGAAGCCAGTTACCTTCGGAAAA
AGAGTTGGTAGCTCTTGATCCGGCAAACAAACCACCGCTGGTAGCGGTGGTTTTTTTGTT
TGCAAGCAGCAGATTACGCGCAGAAAAAAAGGATCTCAAGAAGATCCTTTGATCTTTTCT
ACGGGGTCTGACGCTCAGTGGAACGAAAACTCACGTTAAGGGATTTTGGTCATGAGATT
ATCAAAAAGGATCTTCACCTAGATCCTTTTAAATTAAAAATGAAGTTTTAAATCAATCTAA
AGTATATATGAGTAAACTTGGTCTGACAGTTACCAATGCTTAATCAGTGAGGCACCTATCT
CAGCGATCTGTCTATTTCGTTCATCCATAGTTGCCTGACTCCCCGTCGTGTAGATAACTAC
GATACGGGAGGGCTTACCATCTGGCCCCAGTGCTGCAATGATACCGCGAGACCCACGCTC
ACCGGCTCCAGATTTATCAGCAATAAACCAGCCAGCCGGAAGGGCCGAGCGCAGAAGTG
GTCCTGCAACTTTATCCGCCTCCATCCAGTCTATTAATTGTTGCCGGGAAGCTAGAGTAAG
TAGTTCGCCAGTTAATAGTTTGCGCAACGTTGTTGCCATTGCTACAGGCATCGTGGTGTCA
CGCTCGTCGTTTGGTATGGCTTCATTCAGCTCCGGTTCCCAACGATCAAGGCGAGTTACA
TGATCCCCCATGTTGTGCAAAAAAGCGGTTAGCTCCTTCGGTCCTCCGATCGTTGTCAGA
AGTAAGTTGGCCGCAGTGTTATCACTCATGGTTATGGCAGCACTGCATAATTCTCTTACTG
TCATGCCATCCGTAAGATGCTTTTCTGTGACTGGTGAGTACTCAACCAAGTCATTCTGAG

AATAGTGTATGCGGCGACCGAGTTGCTCTTGCCCGGCGTCAATACGGGATAATACCGCGC
CACATAGCAGAACTTTAAAAGTGCTCATCATTGGAAAACGTTCTTCGGGGCGAAAACTCT
CAAGGATCTTACCGCTGTTGAGATCCAGTTCGATGTAACCCACTCGTGCACCCAACTGAT
CTTCAGCATCTTTTACTTTCACCAGCGTTTCTGGGTGAGCAAAAACAGGAAGGCAAAAT
GCCGCAAAAAAGGGAATAAGGGCGACACGGAAATGTTGAATACTCATACTCTTCCTTTTT
CAATATTATTGAAGCATTTATCAGGGTTATTGTCTCATGAGCGGATACATATTTGAATGTATT
TAGAAAAATAAACAAATAGGGGTTCCGCGCACATTTCCCCGAAAAGTGCCACCTG

The nucleotide sequence of SEQ ID NO.7 is as follows:

ATGAGTCTGGCCATTCCAGTTATGTGCCTGCTGGCAAATACCACGTTCCCCTGCTCCC
AGCCCCCTTGCACACCCTGCTGCTACGAAAAAGAGCCGGAGAAAACCTTGCGCATGCTT
GAAGACAATGTCATGAGCCCCGGGTACTATCAGCTGCTACAAGCATCCTTAACATGTTCT
CCCCGACGCCAGCGGCGCAGTATTAAGGACCACTTCAATGTCTATAAAGCCACAAGACC
GTACCTAGCTCACTGTCCCGACTGTGGAGAAGGGCACTCGTGCCATAGTCCCGTAGCGCT
AGAACGCATCAGAAACGAAGCGACAGACGGGACGTTGAAAATCCAGGTTTCCTTGCAA
ATCGGAATAAAGACGGATGATAGCCATGATTGGACCAAGCTGCGTTATATGGACAATCAC
ATGCCAGCAGACGCAGAGCGGGCCGGGCTATTTGTAAGAACGTCAGCACCGTGCACGAT
TACTGGAACAATGGGACACTTCATTCTGGCCCGATGTCCGAAAGGAGAAACTCTGACGG
TAGGGTTCACTGACGGTAGGAAGATCAGTCACTCATGTACGCACCCATTTCACCATGACC
CTCCTGTGATAGGCCGGGAAAAATTCCATTCCCGACCGCAGCACGGTAGGGAACTACCTT
GCAGCACGTACGCGCAGAGCACCGCTGCAACTGCCGAGGAGATAGAGGTACATATGCCC
CCAGACACCCCAGATCGCACATTAATGTCACAACAGTCCGGCAATGTAAAGATCACAGTC
AATAGTCAGACGGTGCGGTACAAGTGCAATTGTGGTGACTCAAGTGAAGGATTAACCAC
TACAGATAAAGTGATTAATAACTGCAAGGTTGATCAATGCCATGCCGCGGTCACCAATCA
CAAAAAATGGCAGTATAATTCCCCTCTGGTCCCGCGTAATGCTGAATTCGGGGACCGGAA
AGGAAAAGTTCACATTCCATTTCCTCTGGCAAATGTGACATGCAGGGTGCCTAAAGCAA
GAAACCCCACCGTGACGTACGGAAAAAACCAAGTCATCATGTTGCTGTATCCTGACCAC
CCAACGCTCCTGTCCTACAGGAATATGGGAGAAGAACCAAACTATCAAGAAGAGTGGGT
GACGCATAAGAAGGAGATCAGGTTAACCGTGCCGACTGAAGGGCTCGAGGTCACGTGG
GGTAACAATGAGCCGTACAAGTATTGGCCGCAGTTATCCACAAACGGTACAGCCCACGG
CCACCCGCATGAGATAATTCTGTATTATTATGAGCTGTACCCAACTATGACTGTGGTAGTTT
TGTCAGTGGCCTCGTTCATACTCCTGTCGATGGTGGGTGTGGCAGTGGGGATGTGCATGT
GTGCACGACGCAGATGCATTACACCGTACGAACTGACACCAGGAGCTACCGTCCCTTTCC
TGCTTAGCCTAATATGCTGCATTAGAACAGCTAAAGCGGCCACATACCAGGAGGCCGCGG
TATACCTGTGGAACGAGCAGCAGCCTTTATTTTGGCTGCAAGCCCTTATTCCGCTGGCAG

CCCTGATTGTCCTATGTAACTGTCTGAGACTCTTACCATGCTGTTGTAAAATGTTGACTTT
TTTAGCCGTACTGAGCGTCGGTGCCCACACTGTGAGTGCGTACGAACACGTAACAGTGA
TCCCGAACACGGTGGGAGTACCGTATAAGACTCTAGTCAACAGACCGGGCTACAGCCCC
ATGGTATTGGAGATGGAGCTTCTGTCTGTCACCTTGGAACCAACGCTATCGCTTGATTACA
TCACGTGCGAGTATAAAACCGTTATCCCGTCTCCGTACGTGAAATGCTGCGGTACAGCAG
AGTGTAAGGACAAGAGCCTACCTGATTACAGCTGTAAGGTCTTCACCGGCGTCTACCCAT
TCATGTGGGGCGGCGCCTACTGCTTCTGCGACACCGAAAATACGCAATTGAGCGAAGCA
CATGTGGAGAAGTCCGAATCATGCAAAACAGAATTTGCATCAGCATACAGGGCTCATACC
GCATCCGCGTCAGCTAAGCTCCGCGTCCTTTACCAAGGAAATAATATCACTGTAGCTGCTT
ATGCAAACGGCGACCATGCCGTCACAGTTAAGGACGCTAAATTCATAGTGGGGCCAATGT
CTTCAGCCTGGACACCTTTCGACAATAAAATCGTGGTGTACAAAGGCGACGTCTACAACA
TGGACTACCCGCCCTTCGGCGCAGGAAGACCAGGACAATTTGGCGACATCCAAAGTCGC
ACGCCTGAGAGCGAAGACGTCTATGCTAATACACAACTGGTACTGCAGAGACCGTCCGC
GGGTACGGTGCACGTGCCGTACTCTCAGGCACCATCTGGCTTCAAGTATTGGCTAAAAGA
ACGAGGGGCGTCGCTGCAGCACACAGCACCATTTGGCTGTCAAATAGCAACAAACCCGG
TAAGAGCGATGAACTGCGCCGTAGGGAACATGCCTATCTCCATCGACATACCGGACGCGG
CCTTTACCAGGGTCGTCGACGCGCCATCTTTAACGGACATGTCGTGTGAGGTATCAGCCT
GCACCCATTCCTCAGACTTTGGGGGCGTAGCCATCATTAAATATGCAGCCAGTAAGAAAG
GCAAGTGTGCAGTGCACTCGATGACTAACGCCGTCACTATTCGGGAAGCTGAAATAGAA
GTAGAAGGGAACTCTCAGTTGCAAATCTCTTTTTCGACGGCCCTAGCCAGCGCCGAATTT
CGCGTACAAGTCTGTTCTACACAAGTACACTGTGCAGCCGAGTGCCATCCACCGAAAGA
CCATATAGTCAATTACCCGGCGTCACACACCACCCTCGGGGTCCAAGACATTTCCGCTAC
GGCGATGTCATGGGTGCAGAAGATCACGGGAGGTGTGGGACTGGTTGTCGCTGTTGCAG
CACTGATCCTAATCGTGGTGCTATGCGTGTCGTTTAGCAGGCACTAA

The nucleotide sequence of SEQ ID NO.8 is as follows:

ATGTCACTAGTGACCACCATGTGTCTGCTCGCCAATGTGACGTTCCCATGTGCTCAA
CCACCAATTTGCTACGACAGAAAACCAGCAGAGACTTTGGCCATGCTCAGCGTTAACGT
TGACAACCCGGGCTACGATGAGCTGCTGGAAGCAGCTGTTAAGTGCCCCGGAAGGAAA
AGGAGATCCACCGAGGAGCTGTTTAATGAGTATAAGCTAACGCGCCCTTACATGGCCAGA
TGCATCAGATGTGCAGTTGGGAGCTGCCATAGTCCAATAGCAATCGAGGCAGTAAAGAG
CGACGGGCACGACGGTTATGTTAGACTTCAGACTTCCTCGCAGTATGGCCTGGATTCCTC
CGGCAACTTAAAGGGCAGGACCATGCGGTATGACATGCACGGGACCATTAAAGAGATAC
CACTACATCAAGTGTCACTCTATACATCTCGCCCGTGTCACATTGTGGATGGGCACGGTTA
TTTCCTGCTTGCCAGGTGCCCGGCAGGGGACTCCATCACCATGGAATTTAAGAAAGATTC

CGTCAGACACTCCTGCTCGGTGCCGTATGAAGTGAAATTTAATCCTGTAGGCAGAGAACT
CTATACTCATCCCCCAGAACACGGAGTAGAGCAAGCGTGCCAAGTCTACGCACATGATGC
ACAGAACAGAGGAGCTTATGTCGAGATGCACCTCCCGGGCTCAGAAGTGGACAGCAGTT
TGGTTTCCTTGAGCGGCAGTTCAGTCACCGTGACACCTCCTGATGGGACTAGCGCCCTGG
TGGAATGCGAGTGTGGCGGCACAAAGATCTCCGAGACCATCAACAAGACAAAACAGTT
CAGCCAGTGCACAAAGAAGGAGCAGTGCAGAGCATATCGGCTGCAGAACaATAAGTGGG
TGTATAATTCTGACAAACTGCCCAAAGCAGCGGGAGCCACCTTAAAAGGAAAACTGCAT
GTCCCATTCTTGCTGGCAGACGGCAAATGCACCGTGCCTCTAGCACCAGAACCTATGATA
ACCTTCGGTTTCAGATCAGTGTCACTGAAACTGCACCCTAAGAATCCCACATATCTAAcCA
CCCGCCAACTTGCTGATGAGCCTCACTACACGCACGAGCTCATATCTGAACCAGCTGTTA
GGAATTTTACCGTCACCGAAAAAGGGTGGGAGTTTGTATGGGGAAACCACCCGCCGAAA
AGGTTTTGGGCACAGGAAACAGCACCCGGAAATCCACATGGGCTACCGCACGAGGTGAT
AACTCATTATTACCACAGATACCCTATGTCCACCATCCTGGGTTTGTCAATTTGTGCCGCC
ATTGCAACCGTTTCCGTTGCAGCGTCTACCTGGCTGTTTTGCAGATCTAGAGTTGCGTGC
CTAACTCCTTACCGGCTAACACCTAACGCTAGGATACCATTTTGTCTGGCTGTGCTTTGCT
GCGCCCGCACTGCCCGGGCCGAGACCACCTGGGAGTCCTTGGATCACCTATGGAACAAT
AACCAACAGATGTTCTGGATTCAATTGCTGATCCCTCTGGCCGCCTTGATCGTAGTGACTC
GCCTGCTCAGGTGCGTGTGCTGTGTCGTGCCTTTTTTAGTCATGGCCGGCGCCGCAGGCG
CCGGCGCCTACGAGCACGCGACCACGATGCCGAGCCAAGCGGGAATCTCGTATAACACT
ATAGTCAACAGAGCAGGCTACGCACCACTCCCTATCAGCATAACACCAACAAAGATCAA
GCTGATACCTACAGTGAACTTGGAGTACGTCACCTGCCACTACAAAACAGGAATGGATTC
ACCAGCCATCAAATGCTGCGGATCTCAGGAATGCACTCCAACTTACAGGCCTGATGAACA
GTGCAAAGTCTTCACAGGGGTTTACCCGTTCATGTGGGGTGGTGCATATTGCTTTTGCGA
CACTGAGAACACCCAAGTCAGCAAGGCCTACGTAATGAAATCTGACGACTGCCTTGCGG
ATCATGCTGAAGCATATAAAGCGCACACAGCCTCAGTGCAGGCGTTCCTCAACATCACAG
TGGGAGAACACTCTATTGTGACTACCGTGTATGTGAATGGAGAAACTCCTGTGAATTTCA
ATGGGGTCAAAATAACTGCAGGTCCGCTTTCCACAGCTTGGACACCCTTTGATCGCAAAA
TCGTGCAGTATGCCGGGGAGATCTATAATTATGATTTTCCTGAGTATGGGGCAGGACAACC
AGGAGCATTTGGAGATATACAATCCAGAACAGTCTCAAGCTCTGATCTGTATGCCAATAC
CAACCTAGTGCTGCAGAGACCCAAAGCAGGAGCGATCCACGTGCCATACACTCAGGCAC
CTTCGGGTTTTGAGCAATGGAAGAAAGATAAAGCTCCATCATTGAAATTTACCGCCCCTT
TCGGATGCGAAATATATACAAACCCCATTCGCGCCGAAAACTGTGCTGTAGGGTCAATTC
CATTAGCCTTTGACATTCCCGACGCCTTGTTCACCAGGGTGTCAGAAACACCGACACTTT
CAGCGGCCGAATGCACTCTTAACGAGTGCGTGTATTCTTCCGACTTTGGTGGGATCGCCA

CGGTCAAGTACTCGGCCAGCAAGTCAGGCAAGTGCGCAGTCCATGTGCCATCAGGGACT
GCTACCCTAAAAGAAGCAGCAGTCGAGCTAACCGAGCAAGGGTCGGCGACTATCCATTT
CTCGACCGCAtATATCCACCCGGAGTTCAGGCTCCAAATATGCACATCATATGTTACGTGC
AAAGGTGATTGTCACCCCCCGAAAGACCATtTTGTGACACACCCTCAGTATCACGCCCAA
ACATTTACAGCCGCGGTGTCAAAAACCGCGTGGACGTGGTTAACATCCCTGCTGGGAGG
ATCAGCCGTAATTATTATAATTGGCTTGGTGCTGGCTACTATTGTGGCCATGTACGTGCTGA
CCAACCAGAAACATAATTGA

The nucleotide sequence of SEQ ID NO.9 is as follows:

ATGggcgttacaggaatattgcagttacctcgtgatcgattcaagaggacatcattctttctttgggtaattatccttttccaaagaacattttc
catcccacttggagtcatccacaatagcacattacaggttagtgatgtcgacaaactagtttgtcgtgacaaactgtcatccacaaatcaattgagat
cagttggactgaatctcgaagggaatggagtggcaactgacgtgccatctgcaactaaaagatggggcttcaggtccggtgtcccaccaaaggt
ggtcaattatgaagctggtgaatgggctgaaaactgctacaatcttgaaatcaaaaaacctgacgggagtgagtgtctaccagcagcgccagacg
ggattcggggcttcccccggtgccggtatgtgcacaaagtatcaggaacgggaccgtgtgccggagactttgccttccataaagagggtgctttc
ttcctgtatgatcgacttgcttccacagttatctaccgaggaacgactttcgctgaaggtgtcgttgcatttctgatactgccccaagctaagaaggac
ttcttcagctcacaccccttgagagagccggtcaatgcaacggaggacccgtctagtggctactattctaccacaattagatatcaggctaccggtt
ttggaaccaatgagacagagtacttgttcgaggttgacaatttgacctacgtccaacttgaatcaagattcacaccacagtttctgctccagctgaat
gagacaatatatacaagtgggaaaaggagcaataccacgggaaaactaatttggaaggtcaaccccgaaattgatacaacaatcggggagtgg
gccttctgggaaactaaaaaaAacctcactagaaaaattcgcagtgaagagttgtctttcacagttgtatcaaacggagccaaaaacatcagtggt
cagagtccggcgcgaacttcttccgacccagggaccaacacaacaactgaagaccacaaaatcatggcttcagaaaattcctctgcaatggttca
agtgcacagtcaaggaagggaagctgcagtgtcgcatctaacaacccttgccacaatctccacgagtccccaatccctcacaaccaaaccaggt
ccggacaacagcacccataatacacccgtgtataaacttgacatctctgaggcaactcaagttgaacaacatcaccgcagaacagacaacgaca
gcacagcctccgacactccctctgccacgaccgcagccggacccccaaaagcagagaacaccaacacgagcaagagcactgacttcctgga
ccccgccaccacaacaagtccccaaaaccacagcgagaccgctggcaacaacaacactcatcaccaagataccggagaagagagtgccagc
agcgggaagctaggcttaattaccaatactattgctggagtcgcaggactgatcacaggcgggagaagaactcgaagagaagcaattgtcaatg
ctcaacccaaatgcaaccctaatttacattactggactactcaggatgaaggtgctgcaatcggactggcctggataccatatttcgggccagcag
ccgagggaatttacatagaggggctaatgcacaatcaagatggtttaatctgtgggttgagacagctggccaacgagacgactcaagctcttcaa
ctgttcctgagagccacaactgagctacgcaccttttcaatcctcaaccgtaaggcaattgatttcttgctgcagcgatggggcggcacatgccaca
ttctgggaccggactgctgtatcgaaccacatgattggaccaagaacataacagacaaaattgatcagattattcatgattttgttgataaaacccttc
cggaccagggggacaatgacaattggtggacaggatggagacaatggataccggcaggtattggagttacaggcgttataattgcagttatcgct
ttattctgtatatgcaaatttgtcttttagttaattaa

The nucleotide sequence of SEQ ID NO.10 is as follows:

atgccggcagaaaacaagaaagttagattcgaaaatactacttcagacaaagggaaaattcctagtaaagttattaagagctactacggaa
ccatggacattaagaaaataaatgaaggattattggacagcaaaatattaagtgctttcaacacagtaatagcattgcttggatctatcgtgatcatag
tgatgaatataatgatcatccaaaattacacaagatcaacagacaatcaggccgtgatcaaagatgcgttgcagggtatccaacagcagatcaaa

gggcttgctgacaaaatcggcacagagatagggcccaaagtatcactgattgacacatccagtaccattactatcccagctaacattgggctgtta
ggttcaaagatcagccagtcgactgcaagtataaatgagaatgtgaatgaaaaatgcaaattcacactgcctcccttgaaaatccacgaatgtaac
atttcttgtcctaacccactcccttttagagagtataggccacagacagaaggggtgagcaatctagtaggattacctaataatatttgcctgcaaaa
gacatctaatcagatattgaagccaaagctgatttcatacactttacccgtagtcggtcaaagtggtacctgtatcacagacccattgctggctatgg
acgagggctattttgcatatagccacctggaaagaatcggatcatgttcaagaggggtctccaaacaaagaataataggagttggagaggtacta
gacagaggtgatgaagttccttctttatttatgaccaatgtctggaccccaccaaatccaaacaccgtttaccactgtagtgctgtatacaacaatgaa
ttctattatgtactttgtgcagtgtcaactgttggagaccctattctgaatagcacctactggtccggatctctaatgatgacccgtctagctgtgaaac
ccaagagtaatggtgggggttacaatcaacatcaacttgccctacgaagtatcgagaaagggaggtatgataaagttatgccgtatggaccttcag
gcatcaaacagggtgacaccctgtattttcctgctgtaggatttttggtcaggacagagtttaaatacaatgattcaaattgtcccatcacgaagtgtc
aatacagtaaacctgaaaattgcaggctatctatggggattagaccaaacagccattatatccttcgatctggactattaaaatacaatctatcagatg
gggagaaccccaaagttgtattcattgaaatatctgatcaaagattatctattggatctcctagcaaaatctatgattctttgggtcaacctgttttctacc
aagcgtcattttcatgggatactatgattaaatttggagatgttctaacagtcaaccctctggttgtcaattggcgtaataacacggtaatatcaagacc
cgggcaatcacaatgccctagattcaatacatgtccagagatctgctgggaaggagtttataatgatgcattcctaattgacagaatcaattggataa
gcgcgggtgtattccttgacagcaatcagaccgcagaaaatcctgtttttactgtattcaaagataatgaaatactttatagggcacaactggcttctg
aggacaccaatgcacaaaaaacaataactaattgttttctcttgaagaataagatttggtgcatatcattggttgagatatatgacacaggagacaat
gtcataagacccaaactattcgcggttaagataccagagcaatgtacataattaattaatacctgagaggggcccctataactctctacggctaacct
gaatggactacgacatAGTCTAGTCCGCCAAGaccatggtagttatacttgacaagagatgttattgtaatcttttaatattgattttgat
gatctcggagtgtagtgttgggattctacattatgagaaattgagtaaaattggacttgtcaaaggagtaacaagaaaatacaagattaaaagcaatc
ctctcacaaaagacattgttataaaaatgattccgaatgtgtcgaacatgtctcagtgcacagggagtgtcatggaaaattataaaacacgattaaac
ggtatcttaacacctataaagggagcgttagagatctacaaaaacaacactcatgaccttgtcggtgatgtgagattagccggagttataatggcag
gagttgctattgggattgcaaccgcagctcaaatcactgcaggtgtagcactatatgaggcaatgaagaatgctgacaacatcaacaaactcaaaa
gcagcattgaatcaactaatgaagctgtcgttaaacttcaagagactgcagaaaagacagtctatgtgctgactgctctacaggattacattaatact
aatttagtaccgacaattgacaagataagctgcaaacagacagaactctcactagatctggcattatcaaagtacctctctgatttgctttttgtatttgg
ccccaaccttcaagacccagtttctaattcaatgactatacaggctatatctcaggcattcggtggaaattatgaaacactgctaagaacattgggtta
cgctacagaagactttgatgatcttctagaaagtgacagcataacaggtcaaatcatctatgttgatctaagtagctactatataattgtcagggtttatt
ttcctattctgactgaaattcaacaggcctatatccaagagttgttaccagtgagcttcaacaatgataattcagaatggatcagtattgtcccaaatttc
atattggtaaggaatacattaatatcaaatatagagattggattttgcctaattacaaagaggagcgtgatctgcaaccaagattatgccacacctatg
accaacaacatgagagaatgtttaacgggatcgactgagaagtgtcctcgagagctggttgtttcatcacatgttcccagatttgcactatctaacgg
ggttctgtttgccaattgcataagtgttacatgtcagtgtcaaacaacaggcagggcaatctcacaatcaggagaacaaactctgctgatgattgac
aacaccacctgtcctacagccgtactcggtaatgtgattatcagcttagggaaatatctggggtcagtaaattataattctgaaggcattgctatcggt
cctccagtctttacagataaagttgatatatcaagtcagatatccagcatgaatcagtccttacaacagtctaaggactatatcaaagaggctcaacg
actccttgatactgttaatccatcattaataagcatgttgtctatgatcatactgtatgtattatcgatcgcatcgttgtgtatagggttgattacatttatca
gttttatcattgttgagaaaaagagaaacacctacagcagattagaggataggagagtcagacctacaagcagtggggatctctactacattggga
catag

The nucleotide sequence of SEQ ID NO.11 is as follows:

ATGGGACAAATAGTGACATTCTTCCAGGAAGTGCCTCATGTAATAGAAGAGGTGATG
AACATTGTTCTCATTGCACTGTCTGTACTAGCAGTGCTGAAAGGTCTGTACAATTTTGCAA
CGTGTGGCCTTGTTGGTTTGGTCACTTTCCTCCTGTTGTGTGGTAGGTCTTGCACAACCA
GTCTTTATAAAGGGGTTTATGAGCTTCAGACTCTGGAACTAAACATGGAGACACTCAATA
TGACCATGCCTCTCTCCTGCACAAAGAACAACAGTCATCATTATATAATGGTGGGCAATG
AGACAGGACTAGAACTGACCTTGACCAACACGAGCATTATTAATCACAAATTTTGCAATC
TGTCTGATGCCCACAAAAAGAACCTCTATGACCACGCTCTTATGAGCATAATCTCAACTTT
CCACTTGTCCATCCCCAACTTCAATCAGTATGAGGCAATGAGCTGCGATTTTAATGGGGG
AAAGATTAGTGTGCAGTACAACCTGAGTCACAGCTATGCTGGGGATGCAGCCAACCATTG
TGGTACTGTTGCAAATGGTGTGTTACAGACTTTTATGAGGATGGCTTGGGGTGGGAGCTA
CATTGCTCTTGACTCAGGCCGTGGCAACTGGGACTGTATTATGACTAGTTATCAATATCTG
ATAATCCAAAATACAACCTGGGAAGATCACTGCCAATTCTCGAGACCATCTCCCATCGGTT
ATCTCGGGCTCCTCTCACAAAGGACTAGAGATATTTATATTAGTAGAAGATTGCTAGGCAC
ATTCACATGGACACTGTCAGATTCTGAAGGTAAAGACACACCAGGGGGATATTGTCTGAC
CAGGTGGATGCTAATTGAGGCTGAACTAAAATGCTTCGGGAACACAGCTGTGGCAAAAT
GTAATGAGAAGCATGATGAGGAATTTTGTGACATGCTGAGGCTGTTTGACTTCAACAAAC
AAGCCATTCAAAGGTTGAAAGCTGAAGCACAAATGAGCATTCAGTTGATCAACAAAGCA
GTAAATGCTTTGATAAATGACCAACTTATAATGAAGAACCATCTACGGGACATCATGGGAA
TTCCATACTGTAATTACAGCAAGTATTGGTACCTCAACCACACAACTACTGGGAGAACAT
CACTGCCCAAATGTTGGCTTGTATCAAATGGTTCATACTTGAACGAGACCCACTTTTCTGA
TGATATTGAACAACAAGCTGACAATATGATCACTGAGATGTTACAGAAGGAGTATATGGA
GAGGCAGGGGAAGACACCATTGGGTCTAGTTGACCTCTTTGTGTTCAGTACAAGTTTCTA
TCTTATTAGCATCTTCCTTCACCTAGTCAAAATACCAACTCATAGGCATATTGTAGGCAAGT
CGTGTCCCAAACCTCACAGATTGAATCATATGGGCATTTGTTCCTGTGGACTCTACAAAC
AGCCTGGTGTGCCTGTGAAATGGAAGAGATGA

The nucleotide sequence of SEQ ID NO.12 is as follows:

ATGGTTCCTCAGGCTCTCCTGTTTGTACCCCTTCTGGTTTTTCCATTGTGTTTTGGGA
AATTCCCTATTTACACGATACCAGACAAGCTTGGTCCCTGGAGTCCGATTGACATACATCA
CCTCAGCTGCCCAAACAATTTGGTAGTGGAGGACGAAGGATGCACCAACCTGTCAGGGT
TCTCCTACATGGAACTTAAAGTTGGATACATCTTAGCCATAAAAGTGAACGGGTTCACTT
GCACAGGCGTTGTGACGGAGGCTGAAACCTACACTAACTTCGTTGGTTATGTCACAACC
ACGTTCAAAAGAAAGCATTTCCGCCCAACACCAGATGCATGTAGAGCCGCGTACAACTG
GAAGATGGCCGGTGACCCCAGATATGAAGAGTCTCTACACAATCCGTACCCTGACTACCG

CTGGCTTCGAACTGTAAAAACCACCAAGGAGTCTCTCGTTATCATATCTCCAAGTGTGGC
AGATTTGGACCCATATGACAGATCCCTTCACTCGAGGGTCTTCCCTAGCGGGAAGTGCTC
AGGAGTAGCGGTGTCTTCTACCTACTGCTCCACTAACCACGATTACACCATTTGGATGCCC
GAGAATCCGAGACTAGGGATGTCTTGTGACATTTTTACCTCCAGTAGAGGGAAGAGAGC
ATCCAAAGGGAGTGAGACTTGCGGCTTTGTAGATGAAAGAGGCCTATATAAGTCTTTAAA
AGGAGCATGCAAACTCAAGTTATGTGGAGTTCTAGGACTTAGACTTATGGATGGAACATG
GGTCTCGATGCAAACATCAAATGAAACCAAATGGTGCCCTCCCGATAAGTTGGTGAACCT
GCACGACTTTCGCTCAGACGAAATTGAGCACCTTGTTGTAGAGGAGTTGGTCAGGAAGA
GAGAGGAGTGTCTGGATGCACTAGAGTCCATCATGACAACCAAGTCAGTGAGTTTCAGA
CGTCTCAGTCATTTAAGAAAACTTGTCCCTGGGTTTGGAAAAGCATATACCATATTCAACA
AGACCTTGATGGAAGCCGATGCTCACTACAAGTCAGTCGAAACTTGGAATGAGATCCTC
CCTTCAAAAGGGTGTTTAAGAGTTGGGGGGAGGTGTCATCCTCATGTGAACGGGGTGTT
TTTCAATGGTATAATATTAGGACCTGACGGCAATGTCTTAATCCCAGAGATGCAATCATCC
CTCCTCCAGCAACATATGGAGTTGTTGGAATCCTCGGTTATCCCCCTTGTGCACCCCCTGG
CAGACCCGTCTACCGTTTTCAAGGACGGTGACGAGGCTGAGGATTTTGTTGAAGTTCAC
CTTCCCGATGTGCACAATCAGGTCTCAGGAGTTGACTTGGGTCTCCCGAACTGGGGGAA
GTATGTATTACTGAGTGCAGGGGCCCTGACTGCCTTGATGTTGATAATTTTCCTGATGACA
TGTTGTAGAAGAGTCAATCGATCAGAACCTACGCAACACAATCTCAGAGGGACAGGGAG
GGAGGTGTCAGTCACTCCCCAAAGCGGGAAGATCATATCTTCATGGGAATCACACAAGA
GTGGGGGTGAGACCAGACTG

The nucleotide sequence of SEQ ID NO.13 is as follows:

atgaatccaaatcagaagatagtgaccattggatcaatctgtatggtaagtgggataattagcttgatgttacagattgggaacataatctcaa
tatgggtcagtcattcaattcaaacagggaatcaacaccaaactgaaccaatcagaaatactaatttcctcactgagaacactgtagcttcagtaaca
ttagctggcaattcatctctttgccccatcaaaggatgggctgtacacagtaaagacaacagtataaggattgggtccaagggggatgtgtttgtaat
tagagagccgttcatatcatgctcccatctggaatgcagaaccttctttttgactcagggagctttactgaatgacaagcactccaacgggactttca
aagataggagccctcatagaacgctaatgagttgccctataggtgaggctccctctccatataactcgaggtttgagtctgttgcttggtcggcaagt
gcttgccatgatggcaccagttggttgataattggaatttctggtccagacaatggggctgtggcggtattgaaatacaatggcataataacagaca
ctatcaagagttggaggaataacatactgaggacccaagagtctgaatgtgcatgtatgaatggctcttgctttactgttatgacagatggaccaagt
aatgggcaggcatcatataagattttcaaaatagaaaaagggaaagtagttaagtcagtcgaattgaatgcccccaattatcactatgaggaatgct
cctgttatcctgaggctggcgaaatcatatgtgtgtgcagggataattggcatggctcaaacaggccatgggtatctttcaatcagaatttggagtat
caaataggatatatttgcagtggagttttcggtgacaatccacggccgaatgacggtacagggagttgtggaccagtgtcctctaacggggcatat
ggggtaaaagggttctcatttaaatatggcaatggtgtctggatcgggaggaccaaaagcactcattccaggagcggctttgaaatgatttgggat
ccaaacgggtggactggaacggacagtgagttctcgatgaaacaagatatagtagcaataactgattggtcaggatatagcgggagttttgtcca
gcacccagaactgacaggattggattgcatgagaccttgcttctgggttgagttaatcagagggcggcccaaagagagcacaatttggactagtg

ggagcagcatatctttttgtggtgtaaatagtgacactgtgagttggtcttggccagacggtgctgagttgccattcaccattgacaagtag

The nucleotide sequence of SEQ ID NO.14 is as follows:

atggagttgctaatcctcaaagcaaatgcaattaccacaatcctcactgcagtcacattttgttttgcttctggtcaaaacatcactgaagaattt
tatcaatcaacatgcagtgcagttagcaaaggctatcttagtgctctgagaactggttggtataccagtgttataactatagaattaagtaatatcaag
aaaaataagtgtaatggaacagatgctaaggtaaaattgataaaacaagaattagataaatataaaaatgctgtaacagaattgcagttgctcatgc
aaagcacacaagcaacaaacaatcgagccagaagagaactaccaaggtttatgaattatacactcaacaatgccaaaaaaaccaatgtaacatta
agcaagaaaaggaaaagaagatttcttggttttttgttaggtgttggatctgcaatcgccagtggcgttgctgtatctaaggtcctgcacctagaagg
ggaagtgaacaagatcaaaagtgctctactatccacaaacaaggctgtagtcagcttatcaaatggagttagtgttttaaccagcaaagtgttagac
ctcaaaaactatatagataaacaattgttacctattgtgaacaagcaaagctgcagcatatcaaatatagaaactgtgatagagttccaacaaaagaa
caacagactactagagattaccagggaatttagtgttaatgcaggcgtaactacacctgtaagcacttacatgttaactaatagtgaattattgtcatta
atcaatgatatgcctataacaaatgatcagaaaaagttaatgtccaacaatgttcaaatagttagacagcaaagttactctatcatgtccataataaaa
gaggaagtcttagcatatgtagtacaattaccactatatggtgttatagatacaccctgttggaaactacacacatcccctctatgtacaaccaacaca
aaagaagggtccaacatctgtttaacaagaactgacagaggatggtactgtgacaatgcaggatcagtatctttcttcccacaagctgaaacatgta
aagttcaatcaaatcgagtattttgtgacacaatgaacagtttaacattaccaagtgaagtaaatctctgcaatgttgacatattcaaccccaaatatga
ttgtaaaattatgacttcaaaaacagatgtaagcagctccgttatcacatctctaggagccattgtgtcatgctatggcaaaactaaatgtacagcatc
caataaaaatcgtggaatcataaagacattttctaacgggtgcgattatgtatcaaataaaggggtggacactgtgtctgtaggtaacacattatatta
tgtaaataagcaagaaggtaaaagtctctatgtaaaaggtgaaccaataataaatttctatgacccattagtattcccctctgatgaatttgatgcatca
atatctcaagtcaacgagaagattaaccagagcctagcatttattcgtaaatccgatgaattattacataatgtaaatgctggtaaatccaccacaaat
atcatgataactactataattatagtgattatagtaatattgttatcattaattgctgttggactgctcttatactgtaaggccagaagcacaccagtcaca
ctaagcaaagatcaactgagtggtataaataatattgcatttagtaactaa

### Example 3: Cellular Secretion Morphology of Different Viral Envelope Microvesicles Constructed via the VRV System

In order to further determine the properties of microvesicles obtained from the VRV delivery system, we performed electron microscopic observation on different cells secreting microvesicles which are constructed in Example 2. Following the experimental method in Example 2, we co-transfected VEEVrep-EGFP saRNA with expression plasmids constructed by envelope proteins of different viruses. Cells at 48 hours post-transfection were washed 1-2 times with PBS, fixed with 2.5% glutaraldehyde at room temperature for approximately 4 hours, scraped off with a cell scraper, and transferred to Ep tubes using a pipette. Cells or tissues fixed with glutaraldehyde were rinsed 3 times with 0.1 M phosphate buffer (pH 7.4), 15 minutes each time, then fixed at 20°C for 2-3 hours with 1% osmic acid pre-cooled at 4°C (the duration was adjusted based on different samples). They were then rinsed 3 times with 0.1 M phosphate buffer (pH 7.4), 15 minutes each time. Samples were dehydrated with a graded ethanol series (30%, 50%, 70%, 80%, 85%, 90%, 95%, and 100% twice), followed by penetration using a penetrant. The penetrated samples were placed in embedding molds, supplemented with the embedding medium epoxy resin, and polymerized in a 60°C incubator for 48 hours. Ultrathin sections were then performed, with the section thickness typically ranging from 80 to 100 nm.

### Experimental Results

The results showed that viral particles and microvesicle particles were observed on the surfaces of cells infected with wild-type virus TC-83 and VEEVrep-VEEV-E microvesicles, respectively (see FIG. 3A). Among these, the particle size of VEEVrep-VEEV-E microvesicles was similar to that of wild-type virus TC-83, but due to the lack of capsid proteins, VEEV-delC particles lacked a core region similar to that of TC-83 in the center, and their electron density was significantly lower than that of TC-83 virus (FIG. 3A; black arrow indicates the TC-83 core region). Similarly, VEEVrep-RABV-G microvesicles assembled from VEEV replicon-rabies virus envelope glycoprotein G showed clear electron microscopic structures, but their morphology, shape and size differed significantly from those of wild-type RABV (see FIG. 3B). In contrast, electron microscopic structures of VEEVrep-EBOV-GP microvesicles (see Fig. 3C) and VEEVrep-NIV-GF microvesicles (see FIG. 3D) were similar to those of wild-type viruses. Consistent with VEEVrep-VEEV-E, VEEVrep-CHIKV-E microvesicles showed clear electron microscopic structures with a size similar to that of wild-type but lacked a core region formed by capsid proteins (see FIG. 3E). That is, the present disclosure observed the morphological structures of various VRVs constructed by the VRV delivery platform via section electron microscopy and found that they all lack nucleocapsid structures formed by capsid proteins.

### Example 4: Negative Staining Electron Microscopic Structure and Particle Size Observation of VEEVrep-VEEV-E Microvesicles Obtained via the VRV System

In order to further observe the properties of microvesicles obtained via the VRV delivery system, we mass-produced VEEVrep-VEEV-E microvesicles, which exhibited higher delivery efficiency among them. The collected culture supernatant was then concentrated using PEG8000 and subjected to sucrose gradient density centrifugation, and microvesicles were collected at the corresponding bands. Desugaring was performed using Amicon^{®} Ultra-0.5 ultrafiltration tubes, and the resulting pure microvesicle solution was used for subsequent observation by negative staining electron microscopy (TEM) and particle size detection by dynamic light scattering (DLS).

### Experimental Results

As shown in FIG. 4A, under a 120 kV transmission electron microscope, TC-83 exhibited a regular morphology with a compact core, consistent with literature reports. However, VEEVrep-VEEV-E microvesicles showed irregular morphology with a structure similar to cellular microvesicles (FIG. 4A). Additionally, DLS results showed that the average size of VEEVrep-VEEV-E microvesicles was approximately 58 nm (FIG. 4B). That is, the morphological structure of VEEVrep-VEEV-E microvesicles constructed via the VRV delivery platform in the present disclosure differed significantly from that of wild-type virus, with irregular morphology due to the lack of capsid proteins. However, DLS data showed a relatively concentrated particle size distribution.

### Example 5: Negative Staining Electron Microscopic Structure and Particle Size Observation of VEEVrep-hACE2-E Microvesicles Obtained via the VRV System

In the preceding Examples, the present disclosure verified the feasibility of the VRV delivery system for delivering target proteins via saRNA using green fluorescent protein (EGFP). In order to enhance its application potential, this Example replaced EGFP with a therapeutic functional gene and observed the expression of the functional gene.

Optional types of target proteins include, but are not limited to: immunostimulatory genes, receptor genes, suicide genes, gene editing sequences, etc., (see FIG. 5A). Furthermore, we selected the novel coronavirus receptor hACE2 as the target gene to test the delivery capability of the VRV system. The hACE2 sequence is shown in SEQ ID NO.15. The specific experimental process was as follows: firstly, the EGFP gene in the VEEVrep-EGFP saRNA plasmid was replaced with the hACE2 gene through AscI and PacI restriction sites, and in the same way, the corresponding RNA was obtained by reverse transcription. The pcaggs-VEEV-E plasmid and VEEVrep-hACE2-E RNA were transfected according to the method described in Example 2. After co-transfection, the expression of the target proteins at different time points was detected using anti-hACE2 antibodies. Similarly, the supernatant at 48 hours post-infection was collected and infected naive BHK-21 cells to test the delivery efficiency.

### Experimental Results

HACE2 expression was detected by IFA. As shown in FIG. 5B, hACE2 expression could be detected at 24 hours post-transfection. Its expression increases over time, peaking at the 96-hour time point (see FIG. 5B, left). Supernatant at 72 hours post-infection was collected and infected naïve BHK cells, and hACE2 expression was detected at 24 hpi. Results showed that hACE2 was highly expressed with an infectious positive rate of nearly 50% (see FIG. 5B, right). It demonstrates that the VRV delivery system has excellent capability for delivering target genes.

The nucleotide sequence of SEQ ID NO.15 is as follows:

ATGTCAAGCTCTTCCTGGCTCCTTCTCAGCCTTGTTGCTGTAACTGCTGCTCAGTCCA

CCATTGAGGAACAGGCCAAGACATTTTTGGACAAGTTTAACCACGAAGCCGAAGACCTG
TTCTATCAAAGTTCACTTGCTTCTTGGAATTATAACACCAATATTACTGAAGAGAATGTCC
AAAACATGAATAATGCTGGGGACAAATGGTCTGCCTTTTTAAAGGAACAGTCCACACTTG
CCCAAATGTATCCACTACAAGAAATTCAGAATCTCACAGTCAAGCTTCAGCTGCAGGCTC
TTCAGCAAAATGGGTCTTCAGTGCTCTCAGAAGACAAGAGCAAACGGTTGAACACAATT
CTAAATACAATGAGCACCATCTACAGTACTGGAAAAGTTTGTAACCCAGATAATCCACAA
GAATGCTTATTACTTGAACCAGGTTTGAATGAAATAATGGCAAACAGTTTAGACTACAAT
GAGAGGCTCTGGGCTTGGGAAAGCTGGAGATCTGAGGTCGGCAAGCAGCTGAGGCCATT
ATATGAAGAGTATGTGGTCTTGAAAAATGAGATGGCAAGAGCAAATCATTATGAGGACTA
TGGGGATTATTGGAGAGGAGACTATGAAGTAAATGGGGTAGATGGCTATGACTACAGCCG
CGGCCAGTTGATTGAAGATGTGGAACATACCTTTGAAGAGATTAAACCATTATATGAACAT
CTTCATGCCTATGTGAGGGCAAAGTTGATGAATGCCTATCCTTCCTATATCAGTCCAATTG
GATGCCTCCCTGCTCATTTGCTTGGTGATATGTGGGGTAGATTTTGGACAAATCTGTACTC
TTTGACAGTTCCCTTTGGACAGAAACCAAACATAGATGTTACTGATGCAATGGTGGACCA
GGCCTGGGATGCACAGAGAATATTCAAGGAGGCCGAGAAGTTCTTTGTATCTGTTGGTCT
TCCTAATATGACTCAAGGATTCTGGGAAAATTCCATGCTAACGGACCCAGGAAATGTTCA
GAAAGCAGTCTGCCATCCCACAGCTTGGGACCTGGGGAAGGGCGACTTCAGGATCCTTA
TGTGCACAAAGGTGACAATGGACGACTTCCTGACAGCTCATCATGAGATGGGGCATATCC
AGTATGATATGGCATATGCTGCACAACCTTTTCTGCTAAGAAATGGAGCTAATGAAGGATT
CCATGAAGCTGTTGGGGAAATCATGTCACTTTCTGCAGCCACACCTAAGCATTTAAAATC
CATTGGTCTTCTGTCACCCGATTTTCAAGAAGACAATGAAACAGAAATAAACTTCCTGCT
CAAACAAGCACTCACGATTGTTGGGACTCTGCCATTTACTTACATGTTAGAGAAGTGGAG
GTGGATGGTCTTTAAAGGGGAAATTCCCAAAGACCAGTGGATGAAAAAGTGGTGGGAGA
TGAAGCGAGAGATAGTTGGGGTGGTGGAACCTGTGCCCCATGATGAAACATACTGTGAC
CCCGCATCTCTGTTCCATGTTTCTAATGATTACTCATTCATTCGATATTACACAAGGACCCT
TTACCAATTCCAGTTTCAAGAAGCACTTTGTCAAGCAGCTAAACATGAAGGCCCTCTGCA
CAAATGTGACATCTCAAACTCTACAGAAGCTGGACAGAAACTGTTCAATATGCTGAGGCT
TGGAAAATCAGAACCCTGGACCCTAGCATTGGAAAATGTTGTAGGAGCAAAGAACATGA
ATGTAAGGCCACTGCTCAACTACTTTGAGCCCTTATTTACCTGGCTGAAAGACCAGAACA
AGAATTCTTTTGTGGGATGGAGTACCGACTGGAGTCCATATGCAGACCAAAGCATCAAAG
TGAGGATAAGCCTAAAATCAGCTCTTGGAGATAAAGCATATGAATGGAACGACAATGAAA
TGTACCTGTTCCGATCATCTGTTGCATATGCTATGAGGCAGTACTTTTTAAAAGTAAAAAA
TCAGATGATTCTTTTTGGGGAGGAGGATGTGCGAGTGGCTAATTTGAAACCAAGAATCTC
CTTTAATTTCTTTGTCACTGCACCTAAAAATGTGTCTGATATCATTCCTAGAACTGAAGTT

GAAAAGGCCATCAGGATGTCCCGGAGCCGTATCAATGATGCTTTCCGTCTGAATGACAAC
AGCCTAGAGTTTCTGGGGATACAGCCAACACTTGGACCTCCTAACCAGCCCCCTGTTTCC
ATATGGCTGATTGTTTTTGGAGTTGTGATGGGAGTGATAGTGGTTGGCATTGTCATCCTGA
TCTTCACTGGGATCAGAGATCGGAAGAAGAAAAATAAAGCAAGAAGTGGAGAAAATCC
TTATGCCTCCATCGATATTAGCAAAGGAGAAAATAATCCAGGATTCCAAAACACTGATGAT
GTTCAGACCTCCTTT

Each technical feature of the above-mentioned examples can be combined arbitrarily. For the sake of concise description, not all possible combinations of each technical feature in the above examples have been described. However, as long as there is no contradiction in the combination of these technical features, they should be regarded as falling within the scope described in the present description.

The above-mentioned examples only represent several embodiments of the present disclosure, and their descriptions are relatively specific and detailed, but they should not be understood as limiting the scope of the invention patent. It should be pointed out that, for those of ordinary skill in the art, without departing from the concept of the present disclosure, several modifications and improvements can also be made, and these all fall within the protection scope of the present disclosure. Therefore, the protection scope of the present disclosure shall be subject to the appended claims.

## Claims

1. A method for constructing an saRNA microvesicle delivery system, including the following steps:
(1) designing an saRNA expression vector 1 and a membrane protein expression vector 2;
(2) co-transfecting the expression vector 1 and the expression vector 2 into a host cell;
(3) culturing the host cell and isolating to obtain a microvesicle containing saRNA;
wherein the saRNA comprises a target gene sequence;
the replicon in the saRNA expression vector is an alphavirus replicon;
the microvesicle does not comprise a capsid protein.

2. The method according to claim 1, wherein the membrane protein is a viral membrane protein; preferably, the viral membrane protein is selected from the viruses of *Alphavirus, Ebola virus, Arenavirus, Henipavirus, Lyssavirus, Pneumovirus, Alpharetrovirus, Lentivirus, Vesiculovirus, Marburgvirus, Mumpsvirus, Morbillivirus, Influenzavirus A, Influenzavirus B, Influenzavirus C, Hantavirus, Flavivirus, Hepacivirus,* and/or *Rubivirus;*
preferably, the viral membrane protein is selected from CHIKV, VEEV, EBOV, NIV, LASV, RABV, RSV and/or H7N1 viruses.

3. The method according to claim 1 or 2, wherein the target gene is a therapeutic gene or an antigen gene;
preferably, the therapeutic gene is an immunostimulatory gene or a suicide gene;
preferably, the antigen gene is a receptor gene, such as hACE2;
preferably, the nucleotide sequence of hACE2 is as shown in SEQ ID NO.15.

4. The method according to any one of claims 1-3, wherein the host cell is a mammalian cell; preferably, the mammalian cell is selected from a 293 cell, a BHK-21 cell, and/or a VERO cell.

5. A microvesicle delivery vector prepared by the method according to any one of claims 1-4, wherein the delivery vector comprises a microvesicle with a viral membrane protein embedded on the surface and saRNA packaged in the vesicle, the microvesicle does not comprise a capsid protein; preferably, the saRNA also comprises a target gene sequence.

6. The microvesicle delivery vector according to claim 5, wherein the microvesicle is a vesicular body with a bilayer membrane structure assembled from a viral envelope protein and a lipid bilayer.

7. The microvesicle delivery vector according to claim 5 or 6, wherein the particle size of the microvesicle is about 10 nm-1000 nm;
preferably, the particle size of the microvesicle is about 50-100 nm;
preferably, the particle size of the microvesicle is about 58 nm.

8. A cell stably expressing the microvesicle delivery vector according to any one of claims 5-7.

9. Use of the method according to any one of claims 1-4, the microvesicle delivery vector according to any one of claims 5-7 and/or the cell according to claim 8 in the manufacture of a targeted drug.

10. Use of the method according to any one of claims 1-4, the microvesicle delivery vector according to any one of claims 5-7 and/or the cell according to claim 8 in the manufacture of a vaccine preparation.
